# EUROPEAN PATENT SPECIFICATION

(11) **EP 4 481 392 B1**
(45) Date of publication and mention of the grant of the patent: **30.07.2025**
(21) Application number: 23205522.8
(22) Date of filing: 24.10.2023
(51) Int. Cl.: G01N 35/00, G01N 35/10, G01N 35/02, G01N 35/04, C12N 15/10

(54) **PIPETTING ROBOT AND CONTROL METHOD**
PIPETTIERROBOTER UND STEUERUNGSVERFAHREN
ROBOT DE PIPETAGE ET PROCÉDÉ DE COMMANDE

(30) Priority: 21.06.2023 CN 202310738210
(43) Date of publication of application: 25.12.2024
(73) Proprietor: Changsha Imadek Intelligent Technology Co., Ltd., Hunan (CN)
(72) Inventor: YAO, Hao, Changsha (CN); DUAN, Lijiang, Changsha (CN)
(74) Representative: Secerna LLP

(56) References cited:
- CN-A- 106 367 311
- US-A1- 2002 098 115
- US-A1- 2006 210 435
- US-A1- 2020 025 782

## Description

### TECHNICAL FIELD

The present invention relates to the field of biological material analysis, in particular to a pipetting robot and a control method.

### BACKGROUND

Nucleic acid detection is an important technical means in medicine and biology. Among the existing nucleic acid extraction methods, the magnetic bead-based method for nucleic acid extraction is a common method for extracting nucleic acids, which has the advantages of rapidity, simplicity, and accuracy, and can be used to extract DNA, RNA, and other nucleic acid substances from biological materials. However, at present, the extraction process is mainly performed through manual operations, so sanitary conditions in the entire extraction environment need to be maintained properly so as to avoid any contamination of the samples. At the same time, after the sample adding for the sample placement plate is completed, it is also necessary to manually transfer the sample placement plate to the magnetic attraction device to complete the separation of nucleic acids from the waste liquid. In this process, extreme care is required in transferring the sample placement plate because of the need to prevent the liquid from spilling out of the sample adding hole, which makes the whole process extremely cumbersome and time-consuming, and also makes it easy to contaminate the test environment in the event of spilling.

US 2006/210435 A1 provides a bar-code driven, completely automated, microplate-based analyzer system for performing chemical, biochemical or biological assays. The analyzer is a modular, bench-top instrument that compactly integrates subsystems for sample dispensing, liquid handling, microplate transport, thermal incubation, vortexing, solid phase separation and optical reading. An internal processor is included for automating the instrument, and a user interface to facilitate communication with the operator via a touch-sensitive liquid-crystal display (LCD), and communicating with a remote network via multiple protocols. The analyzer includes firmware resident within the processing system and the user interface allows the operator to select pre-defined assay batch protocols and the user interface is configured in such as way so as to restrict an operator from programming the firmware.

In US 2020/025782 A1, systems and methods for automated cell processing of biological samples, such as cells for use in cell therapy and regenerative medicine. Systems for automated processing of batches derived from biological samples comprise: a closed and sterile enclosure; a plurality of reagent containers; at least one reagent dispenser; a quality control module for analyzing at least one characteristic of a batch; a harvesting module; a robotic module; and a control unit (CU) communicatively coupled to the at least one reagent dispenser, the quality control module, the harvesting module and the robotic module for controlling the automatic processing of batches. The automatic processing may be executable without handling by a human operator. The system may be configured to automatically process the plurality of batches without cross-contamination between batches, e.g., under GMP conditions.

**In** US 2002/098115 A1, a locator bed has nests where sample well containing plates are held and positioned for accessing by probes moved by an X-Y-Z positioning system. Each nest includes stop posts at two sides of a rectangular base of a plate, and biasing posts at the opposed two sides. The stop posts and the biasing posts have tapered upper guide portions for guiding a descending plate into position and cylindrical lower portions receiving the base of the plate in a seated position. The stop posts are located on the surface of the locator bed by conical seat portions. The biasing posts are placed over studs fastened to the bed surface, and hoop springs are captured between the studs and the biasing posts.

CN 106367311 A provides a nucleic acid extraction system and a using method thereof. Transverse and longitudinal motion of a liquid moving mechanism are achieved through a transverse motion mechanism and a longitudinal motion mechanism, thereby implementing a motion between a gun head abandoning mechanism, a magnetic absorbing mechanism and a gun head mechanism of the liquid moving mechanism. The gun head abandoning mechanism is configured to discharge waste liquid and abandon a gun head. The magnetic absorbing mechanism is configured to place the gun head and all samples. The liquid moving mechanism moves among the gun head abandoning mechanism. Then, the magnetic absorbing mechanism and the gun head mechanism are configured to extract the gun head, inject the samples, extract nucleic acid and discharge the waste liquid.

### SUMMARY

The present invention aims to solve at least one of the technical problems in the existing technology. In view of this, the present invention proposes a pipetting robot, which can address the problems of troublesome operation, long operation time and easy contamination in the nucleic acid separation process.

The present invention further proposes a pipetting robot control method.

The features of the pipetting robot and the pipetting robot control method according to embodiments of the present disclosure are set out in the appended set of claims.

Additional features and advantages of the present invention will be set forth in the subsequent description, and in part will become apparent from the description, or may be learned by practice of the present invention.

### BRIEF DESCRIPTION OF DRAWINGS

The above and/or additional aspects and advantages of the present invention will become apparent and readily understood from the description of embodiments made in conjunction with the following accompanying drawings, in which:
FIG. 1 is a structural diagram of a pipetting robot as provided in an embodiment of the present invention (the sealing shell is not shown);
FIG. 2 is a structural diagram of a pipetting robot as provided in an embodiment of the present invention from one viewpoint;
FIG. 3 is a structural diagram of a pipetting robot as provided in an embodiment of the present invention from another viewpoint;
FIG. 4 is a structural diagram of the combination of an inline sample adding pump with a plate moving wrench as provided in an embodiment of the present invention (the shell is not shown);
FIG. 5 is a structural diagram of the rest of the combined test operation mechanisms as provided in an embodiment of the present invention (the shell is not shown);
FIG. 6 is an overall structural schematic diagram of a magnetic attraction module as provided in an embodiment of the present invention;
FIG. 7 is an exploded schematic diagram of a magnetic attraction module as provided in an embodiment of the present invention;
FIG. 8 is a cross-sectional diagram of a magnetic attraction module as provided in an embodiment of the present invention;
FIG. 9 is a schematic diagram of a limiting mechanism as provided in an embodiment of the present invention;
FIG. 10 is a schematic diagram of an abutting structure when it is at a third position as provided in an embodiment of the present invention;
FIG. 11 is a schematic diagram of an abutting structure when it is at a fourth position as provided in an embodiment of the present invention;
FIG. 12 is a disassembly schematic diagram of an adapter assembly as provided in an embodiment of the present invention;
FIG. 13 is an exploded schematic diagram of an adapter base as provided in an embodiment of the present invention;
FIG. 14 is an enlarged diagram at A in FIG. 11; and
FIG. 15 is a flowchart of a pipetting robot control method as provided in an embodiment of the present invention.

### DETAILED DESCRIPTION

Embodiments of the present invention will be described in detail below. Examples of the embodiments are illustrated in the accompanying drawings, where the same or like reference numerals throughout the figures indicates the same or like elements having the same or like functions. The embodiments described below with reference to the accompanying drawings are exemplary and are intended only to explain the present invention instead of being construed as limiting the present invention.

In the description of the present invention, if there is a description of "first", "second", etc., it is used only for the purpose of distinguishing the technical features, and is not to be understood as indicating or implying relative importance or implicitly specifying the number of technical features indicated or implicitly specifying the sequential relationship of the technical features indicated.

In the description of the present invention, it should be understood that, descriptions relating to orientation, for example, orientation or positional relationships indicated by "up", "down", etc., are based on the orientation or positional relationships shown in the accompanying drawings, and are only for the purpose of facilitating the description of the present invention and simplifying the description, rather than indicating or implying that the apparatus or element referred to must have a specific orientation or be constructed and operated in a specific orientation, and therefore cannot be construed as limiting the present invention.

In the description of the present invention, it is to be noted that, unless otherwise explicitly defined, the terms such as "provided", "mounted", and "connected" should be construed in a broad sense, and those of ordinary skills in the art can determine the specific meanings of the above terms in the present invention in a rational way in conjunction with the specific contents of the technical schemes.

A clear and complete description of the technical schemes of the present invention will be given below in conjunction with the accompanying drawings, and it is obvious that the embodiments described below are a part of the embodiments of the present invention and not all of the embodiments.

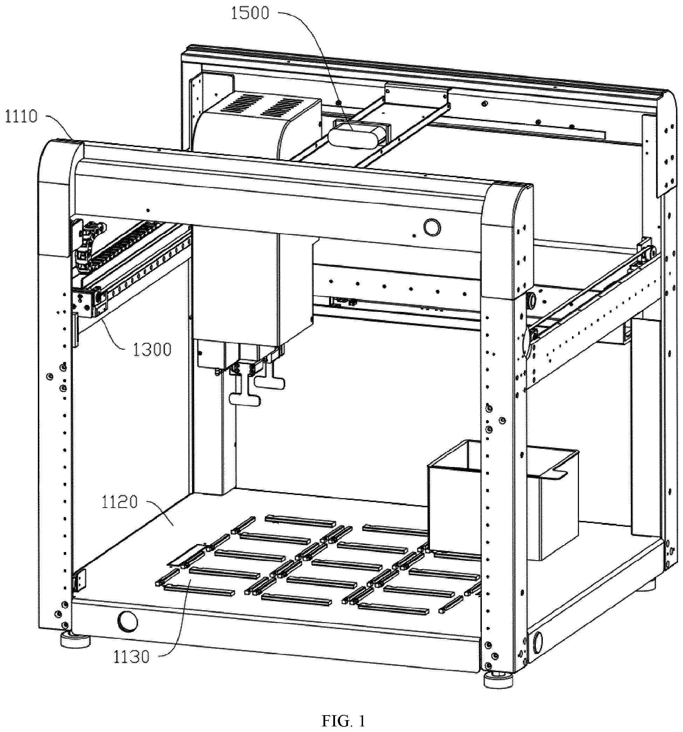

Referring to FIG. 1 through FIG. 3, the pipetting robot includes: a sealing body 1100, an air filtration assembly 1200, a multi-directional movement assembly 1300, a sample adding pump, a plate moving wrench 1410, a camera assembly 1500, and a display and control assembly 1600, wherein:
the sealing body 1100 includes a mounting frame 1110 and a sealing shell wrapped around the mounting frame 1110, wherein the mounting frame 1110 is provided with a placement platform 1120 at a bottom thereof, the placement platform 1120 is provided with a plurality of plate positions 1130, the plurality of the plate positions 1130 are configured for at least placing a reagent unit, a sample unit, a magnetic attraction module, and a sample placement plate 2500; wherein the sample unit is configured for placing a nucleic acid sample to be tested, and the reagent unit is configured for placing a nucleic acid test auxiliary material, the nucleic acid test auxiliary material including magnetic particles; the sample placement plate 2500 has a plurality of sample placement holes 2501; and the magnetic attraction module includes a driving mechanism 2400 and a plurality of liftable magnetic attraction members 2301 driven by the driving mechanism 2400, and the magnetic attraction module is configured for placing the sample placement plate 2500 and is capable of raising the magnetic attraction members 2301 to a first position close to the sample placement plate 2500 and lowering the magnetic attraction members 2301 to a second position away from the sample placement plate 2500;
the air filtration assembly 1200 is provided on the sealing shell and configured for filtering and cleaning air entering and leaving the sealing shell;
the multi-directional movement assembly 1300 is provided on the mounting frame 1110, the multi-directional movement assembly 1300 having a free-moving end, wherein the free-moving end is capable of performing multi-directional movement above the placement platform 1120;
the sample adding pump is provided on the free-moving end, the sample adding pump is configured for injecting and suctioning a liquid;
the plate moving wrench 1410 is provided on the free-moving end, the plate moving wrench 1410 is configured for moving the sample placement plate 2500;
the camera assembly 1500 is provided within the sealing shell and configured for collecting image information for an interior of the sealing shell; and
the display and control assembly 1600 is electrically connected separately to the driving mechanism 2400, the air filtration assembly 1200, the multi-directional movement assembly 1300, the sample adding pump, the plate moving wrench 1410, and the camera assembly 1500.

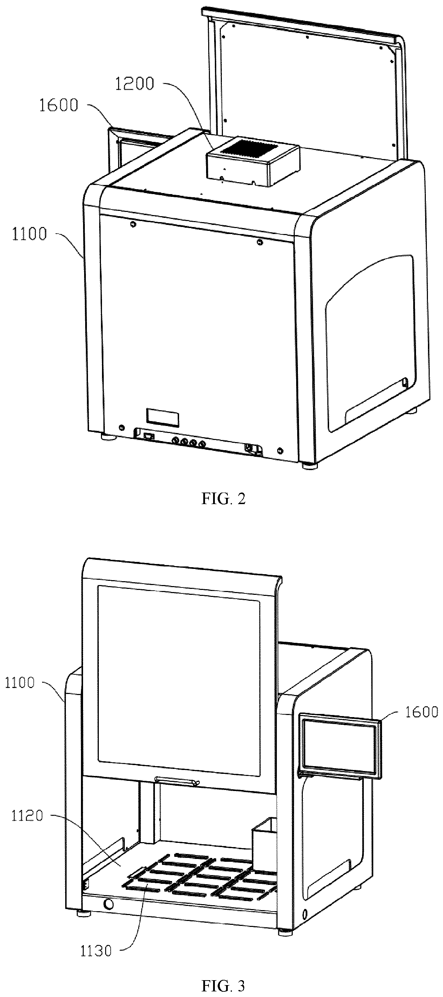

As shown in FIG. 1, the mounting frame 1110 is provided with a placement platform 1120 at the bottom, and the placement platform 1120 is provided with a plurality of plate positions 1130, wherein each of the plate positions 1130 can realize the placement of a functional module (e.g., a reagent unit, a sample unit, a magnetic attraction module, a sample placement plate 2500, and so on). The mounting frame 1110 is provided with a multi-directional movement assembly 1300 at the top, and the multi-directional movement assembly 1300 is provided with a connecting mechanism at the free-moving end, so that the mounting of extension tools such as the sample adding pump and the plate moving wrench 1410 can be realized, and the tools such as the plate moving wrench 1410 and the sample adding pump can move freely within the upper space of the placement platform 1120 by means of the free-moving end, so as to enable operations such as liquid adding, liquid suctioning, and plate moving to be carried out at any position. The mounting frame 1110 is provided with a camera assembly 1500 at the top, and the camera assembly 1500 can be used to realize image capture of the item on the placement platform 1120, which facilitates the subsequent determination of the type of the item and the position in which the item is located, thus providing a basis for the subsequent realization of automated operation. Finally, the sealing shell is used to seal the mounting frame 1110 to obtain a sealed space, which eliminates the influence of external factors on the internal sealed space; and the air filtration assembly 1200 can be used to filter and sterilize the air inside the sealed space, which further ensures the sterile state inside the module shell 2101, thereby reducing the contamination of the nucleic acid sample. In some embodiments, as shown in FIGS. 2 and 3, one side of the sealing shell is provided to be in a sliding structure so as to realize the opening of the sealing shell by means of up-and-down sliding or left-and-right sliding, thus allowing the tester to place the material and to take the separated sample.

The magnetic attraction module can be mounted through the plate position 1130, and the magnetic attraction module can adopt a structure with a built-in power supply in a wireless electrical connection with the display and control assembly 1600, so that the control of the raising and lowering of the magnetic attraction members 2301 can be realized under the control of the display and control assembly 1600. It is to be noted that the magnetic attraction module can also adopt a structure with an external power supply connected to the display and control assembly 1600 through the power supply line and the communication line, wherein the power supply line can be wired separately. **In** addition, after the magnetic attraction members 2301 in the magnetic attraction module adopt a structure of raising and lowering settings, the magnetic attraction members 2301 can be controlled to be lowered before the magnetic particles adsorb the nucleic acid composition, so as to avoid influence of the magnetic force in the magnetic attraction members 2301 on the magnetic particles in the sample adding holes 2501, whereas after the adsorption is completed, the magnetic attraction members 2301 can be controlled to be raised so as to attract the magnetic particles, and then the sample adding pump can be operated to suction the waste liquids from the sample adding holes, thus realizing the nucleic acid separation.

**In** order to better understand the advantages of this embodiment in working, the automated working process of nucleic acid separation by the pipetting robot in this embodiment is described here.

Before starting the work, the plate moving wrench 1410 and the sample adding pump are first mounted on the free-moving end by the tester, and the reagent unit, the sample unit, the magnetic attraction module, the sample placement plate 2500, the sample adding probe box, and the waste probe box are placed on different plate positions 1130 in the placement platform 1120. It can be understood that the numbers of reagent units, sample units, and sample placement plates 2500 can be flexibly adjusted according to the actual needs and the plate positions 1130 on the placement platform 1120, so as to realize the purpose of completing multiple extractions by one placement.

At the end of the preparation process, the sealing shell is closed. A working component currently connected to the free-moving end is determined through the display and control assembly 1600, and a test operation strategy for a specific test is selected, for example, a nucleic acid separation strategy. Afterwards, the nucleic acid separation can be automatically performed by the pipetting robot. The specific separation process is as follows.

The position information for the reagent unit, the sample unit, the magnetic attraction module, the sample placement plate 2500, the sample adding probe box, and the waste probe box is determined by continuously acquiring image information on the placement platform 1120 via the camera assembly 1500. The plate moving wrench 1410 is controlled to move the sample placement plate 2500 onto the magnetic attraction module, and the magnetic attraction members 2301 in the magnetic attraction module are controlled to be lowered. The sample adding pump is controlled to go to the sample adding probe box to suction a sample adding probe and, after the sample adding probe is connected, go to the sample unit to suction a nucleic acid sample to be tested and transfer the nucleic acid sample to be tested to a sample placement hole 2501 of the sample placement plate 2500, and then the sample adding pump is controlled to release the sample adding probe to the waste probe box, wherein the step needs to be repeated several times if there are a plurality of nucleic acid samples to be tested. After the adding of the nucleic acid sample to be tested is completed, the sample adding pump is controlled to go to the sample adding probe box to suction a sample adding probe and, after the sample adding probe is connected, go to the reagent unit to suction a nucleic acid test auxiliary material and transfer the nucleic acid test auxiliary material to a sample placement hole 2501 of the sample placement plate 2500, wherein the number of times for performing this step corresponds to the number of times for the foregoing adding of the nucleic acid sample to be tested, and it is to be noted that the nucleic acid test auxiliary material includes magnetic particles to enable adsorption of nucleic acids in the nucleic acid sample to be tested. After the mixing is completed, the magnetic attraction members 2301 in the magnetic attraction module are controlled to be raised, so as to attract the magnetic particles that have adsorbed nucleic acids into the sample placement hole 2501, and after that, the sample adding pump is used to go to the sample adding probe box to suction a sample adding probe so as to suction the waste liquid of the nucleic acid sample to be tested from which the nucleic acids have been attracted away, thereby realizing nucleic acid separation. It is to be noted that the suctioned waste liquid can be centrally placed in a special waste liquid container provided on the placement platform 1120 for subsequent processing. At the same time, when transferring the waste liquid, it is also necessary to replace the sample adding probe every time the waste liquid in the sample placement hole 2501 is transferred, so as to avoid contamination between the samples.

As shown in FIG. 1, in some embodiments, the multi-directional movement assembly 1300 includes an X-directional movement assembly, a Y-directional movement assembly, and a Z-directional movement assembly, wherein the X-directional movement assembly is movably provided at the top of the mounting frame 1110, the Y-directional movement assembly is movably provided at the lower end of the X-directional movement assembly, and the Z-directional movement assembly is movably provided at the lower end of the Y-directional movement assembly, and the Z-directional movement assembly is provided with a connecting mechanism at the lower end, which can be configured for mounting an extension tool such as a plate moving wrench 1410, a sample adding pump, and the like. After the mounting of the work assembly is completed, the movement of the work assembly such as the plate moving wrench 1410 and the sample adding pump can be realized by controlling the work of the X-directional movement assembly, the Y-directional movement assembly, and the Z-directional movement assembly, so that the manipulation of various items on the placement platform 1120 can be realized. It can be understood that, for the X-directional movement assembly, the Y-directional movement assembly and the Z-directional movement assembly, electric assemblies can be selected, or pneumatic assemblies or hydraulic assemblies can be selected.

In some embodiments, the air filtration assembly 1200 includes air filtration wool, a fan, and an ultraviolet lamp, and can ensure that a sterile environment is achieved inside by constantly sterilizing the air inside the sealing shell.

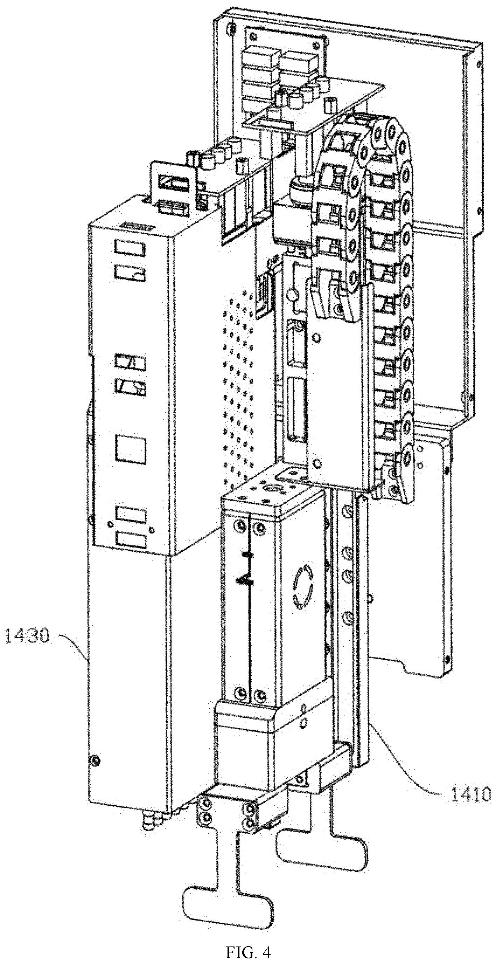

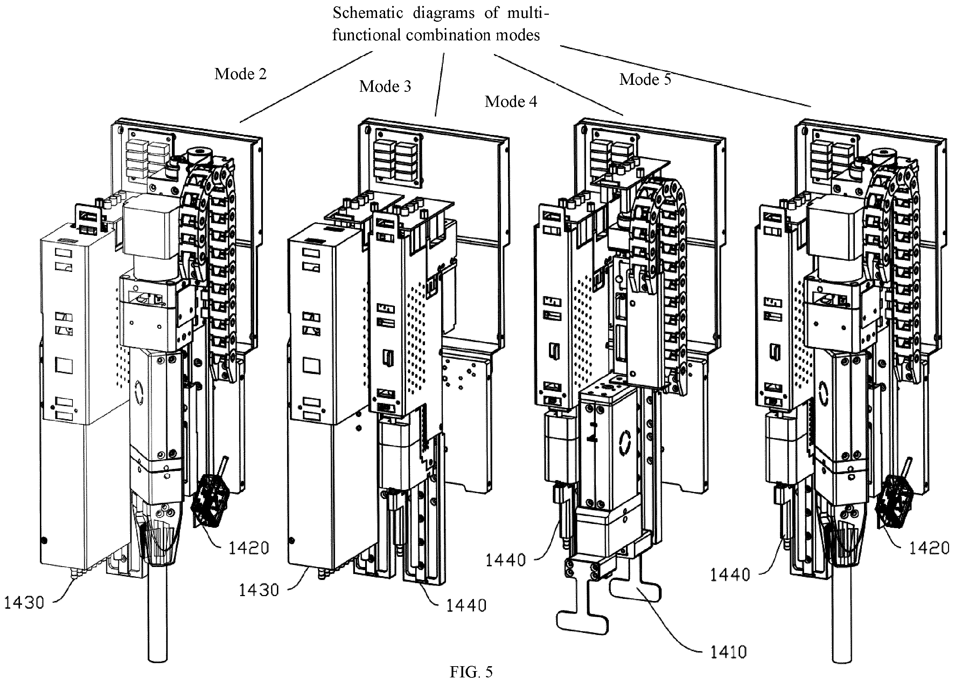

Referring to FIGS. 4 and 5, in some embodiments, the pipetting robot further includes a lid opening grasper 1420 and an oscillation module electrically connected to the display and control assembly 1600. The sample adding pump includes an inline sample adding pump 1430 and a single probe sample adding pump 1440. The display and control assembly 1600 is preset with a plurality of test operation strategies, wherein each of the test operation strategies corresponds to one combined test operation mechanism, the combined test operation mechanism including at least a combination of the inline sample adding pump 1430 with the plate moving wrench 1410, a combination of the single probe sample adding pump 1440 with the plate moving wrench 1410, a combination of the inline sample adding pump 1430 with the lid opening grasper 1420, a combination of the single probe sample adding pump 1440 with the lid opening grasper 1420, and a combination of the inline sample adding pump 1430 with the single probe sample adding pump 1440, and each of the test operation strategies is configured for controlling a corresponding combined test operation mechanism among the combined test operation mechanisms to complete a test operation, and the display and control assembly 1600 is configured for selecting a corresponding one of the test operation strategies according to the combined test operation mechanism.

The inline sample adding pump 1430 and the single probe sample adding pump 1440 are commonly used sample adding pumps, the oscillation module is configured for test trials where adequate mixing is required, and the lid opening grasper 1420 can be used to open the lid of a container with a sealing lid, and in combination with some of the aforementioned module components, they are substantially enough to meet the requirements of most tests. This embodiment proposes a working mode of presetting test operation strategies, according to which the developer can preset some commonly used test operation strategies in the display and control assembly 1600 in advance, wherein each of the test operation strategies is to complete a kind of test trial based on a combined test operation mechanism, and when the user determines that a certain test needs to be performed, the user can load the desired combined test operation mechanism on the automated moving end, and then select the corresponding test operation strategy, so that the pipetting robot can complete the test on its own, for example, the nucleic acid separation test described above.

Here, a brief introduction is provided for the use manner of several common combinations such as the combination of the inline sample adding pump 1430 with the plate moving wrench 1410, the combination of the single probe sample adding pump 1440 with the plate moving wrench 1410, the combination of the inline sample adding pump 1430 with the lid opening grasper 1420, the combination of the single probe sample adding pump 1440 with the lid opening grasper 1420, and the combination of the inline sample adding pump 1430 with the single probe sample adding pump 1440.

FIG. 4 illustrates the structure of the combination of the inline sample adding pump 1430 with the plate moving wrench 1410 after removal of the module shell 2101 (for the combined test operation mechanism of the inline sample adding pump 1430 and the plate moving wrench 1410 in FIG. 1, which is connected to the multi-directional movement assembly 1300, the shell is not removed), i.e., combination mode 1, wherein this combination can be used to conduct nucleic acid separation tests and also other tests. The functions of the inline sample adding pump 1430 and the single probe sample adding pump 1440 are similar. Therefore, for the working mode of the structure of the combination of the single probe sample adding pump 1440 with the plate moving wrench 1410 (as shown in mode 4 in FIG. 5), it can be realized by directly referring to the structure of the combination of the inline sample adding pump 1430 with the plate moving wrench 1410. For a general operating procedure of the inline sample adding pump 1430 plus the plate moving wre, reference can be made to the following description.

First, the front housing in the sealing shell is slidably opened, and the reagent unit, the sample unit, the sample placement plate 2500, the waste probe box, the sample adding probe box, the detection equipment, and the oscillation module are placed on the plate positions of the placement platform 1120, and then with the cooperation of the X-directional movement assembly, the Y-directional movement assembly, and the Z-directional movement assembly, the inline sample adding pump 1430 is moved to the region where the sample adding probe box is located to suction a sample adding probe, and then is moved to the region where the sample unit is located to suction the liquid in the sample tube, after which the inline sample adding pump 1430 places the suctioned liquid into a sample placement hole 2501 in the sample placement plate 2500, and then moves the sample adding probe consumable to the waste probe box for disengagement. The inline sample adding pump 1430 is moved to the region where the sample adding probe box is located to suction a sample adding probe, and then moved to the region where the reagent unit is located to suction the reagent, and then adds the suctioned reagent to the sample placement hole 2501 of the sample placement plate 2500 that contains the sample, after which the inline sample adding pump 1430 moves the sample adding probe to the waste probe box for disengagement. The plate moving wrench is moved to grasp the sample placement plate 2500 of the placement platform 1120, and places the sample placement plate 2500 into the oscillation module so as to perform oscillatory mixing according to the test requirements. The inline sample adding pump 1430 is moved to the region where the sample adding probe box is located to suction a sample adding probe and moved to the oscillation module to suction the mixed sample, and then sends the mixed sample to the desired detection equipment for detection, and finally the inline sample adding pump 1430 moves the sample adding probe consumable to the waste probe box for disengagement.

As shown in FIG. 4, the illustrated mode 2 is the structure of the combination of the inline sample adding pump 1430 with the lid opening grasper 1420 after the removal of the module shell 2101, wherein this combination can be configured for a test that requires lid opening. The functions of the inline sample adding pump 1430 and the single probe sample adding pump 1440 are similar. Therefore, for the working mode of the structure of the combination of the single probe sample adding pump 1440 with the lid opening grasper 1420 (as shown in mode 5 in FIG. 4), it can be realized by directly referring to the structure of the combination of the inline sample adding pump 1430 with the lid opening grasper 1420. For a general operating procedure of the inline sample adding pump 1430 plus the lid opening grasper 1420, reference can be made to the following description.

First, the front housing in the sealing shell is opened, and the reagent unit, the sample unit, the sample placement plate 2500, the waste probe box, the sample adding probe box, the clamping module, the detection equipment, and other functional modules required for the test are placed on the plate positions 1130 of the placement platform 1120, and then with the cooperation of the X-directional movement assembly, the Y-directional movement assembly, and the Z-directional movement assembly, the inline sample adding pump 1430 is moved to the region where the sample adding probe box is located to suction a sample adding probe; afterwards, the lid opening grasper 1420 is moved to grasp the sample tube in the sample unit for rotational scanning so as to record the sample information, and then to move the sample tube into the clamping module, the clamping module clamps the sample tube, the lid opening grasper 1420 opens the lid of the sample tube, and the inline sample adding pump 1430 suctions liquid from the sample tube, and then the lid opening grasper 1420 closes the lid of the sample tube and the inline sample adding pump 1430 is moved to place the suctioned liquid into the sample placement plate 2500, after which the inline sample adding pump 1430 moves the sample adding probe to the waste probe box for disengagement. The inline sample adding pump 1430 is moved to the region where the sample adding probe box is located to suction a sample adding probe, and then moved to the region where the reagent unit is located to suction the reagent, and then adds the suctioned reagent to the sample placement plate 2500 that contains the sample for mixing, after which the mixed sample is sent to the desired detection equipment for detection, and finally the inline sample adding pump 1430 moves the sample adding probe to the waste probe box for disengagement.

As shown in FIG. 5, the illustrated mode 3 is the structure of the combination of the inline sample adding pump 1430 with the single probe sample adding pump 1440 after the removal of the module shell 2101, wherein this combination can be configured for a test that requires multiple pipetting. For the general operating procedure, reference is made to the following description.

First, the front housing in the sealing shell is opened, and the reagent unit, the sample unit, the sample placement plate 2500, the waste probe box, the sample adding probe box, the detection equipment, and other functional modules required for the test are placed on the plate positions 1130 of the placement platform 1120, and then with the cooperation of the X-directional movement assembly, the Y-directional movement assembly, and the Z-directional movement assembly, the inline sample adding pump 1430 is moved to the region where the sample adding probe box is located to suction a sample adding probe, and then is moved to the region where the sample unit is located to suction the liquid in the sample tube, after which the inline sample adding pump 1430 places the suctioned liquid into a sample placement hole 2501 in the sample placement plate 2500, and then moves the sample adding probe consumable to the waste probe box for disengagement. The single probe sample adding pump 1440 is moved to the region where the sample adding probe box is located to suction a sample adding probe, and then moved to the region where the reagent unit is located to suction the reagent, and then adds the suctioned reagent to the sample placement hole 2501 of the sample placement plate 2500 that contains the sample, after which the single probe sample adding pump 1440 moves the sample adding probe to the waste probe box for disengagement. The above steps are repeated until all the processes requiring pipetting configurations are completed, and then the mixed sample is suctioned through the inline sample adding pump 1430 to the detection equipment for detection.

**In** some embodiments, the magnetic attraction module includes: a mounting base 2100, a sample placement plate base 2200, a magnetic attraction base 2300, and a driving mechanism 2400, wherein:
the sample placement plate base 2200 is provided on the mounting base 2100, the sample placement plate base 2200 is configured for mounting the sample placement plate 2500;
the magnetic attraction base 2300 is liftably mounted to the mounting base 2100 and located below the sample placement plate base 2200, the magnetic attraction base 2300 is provided with the magnetic attraction members 2301, the magnetic attraction base 2300 is capable of being raised to the first position of bringing the magnetic attraction members 2301 close to the sample placement plate 2500 and being lowered to the second position of bringing the magnetic attraction members 2301 away from the sample placement plate 2500; and
the driving mechanism 2400 is provided on the mounting base 2100 and in transmission connection with the magnetic attraction base 2300, the driving structure 3400 is electrically connected to the display and control assembly 1600 for controlling the raising and lowering of the magnetic attraction base 2300.

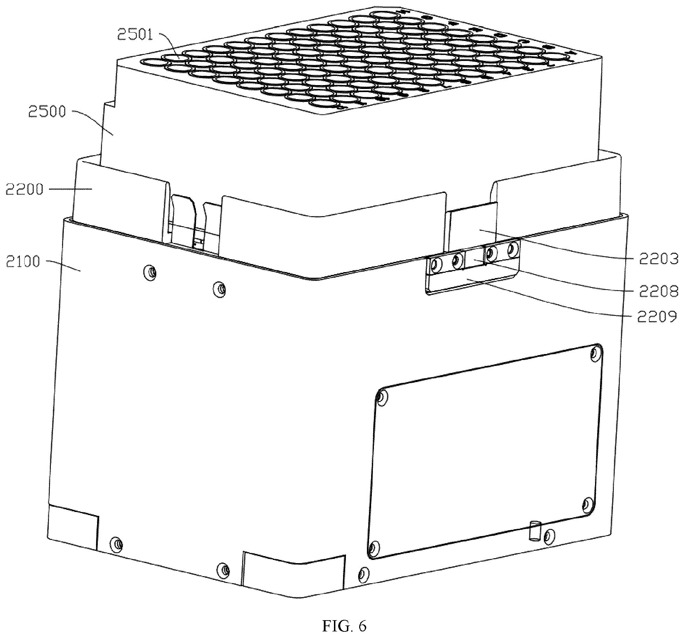

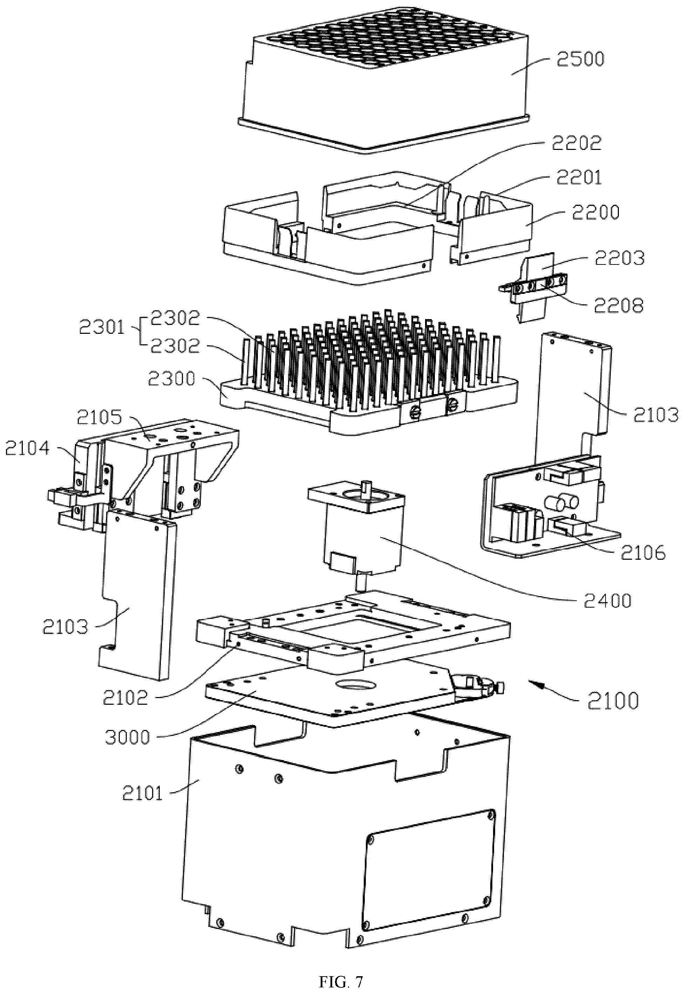

**In** the present invention, referring to FIGS. 6 and 7, when it is necessary to extract nucleic acids by the magnetic bead-based method, the magnetic attraction base 2300 is controlled by the driving mechanism 2400 to be lowered, so that the magnetic attraction members 2301 are away from the sample placement plate 2500, after which the sample and the special magnetic particles can be added to the sample placement hole 2501 of the sample placement plate 2500. Because the magnetic attraction members 2301 are away from the sample placement plate 2500, the magnetic attraction members 2301 will not affect the mixing and the adsorption between the nucleic acids in the sample and the magnetic particles. After the nucleic acids in the sample and the magnetic particles are mutually mixed and adsorbed, the driving mechanism 2400 controls the magnetic attraction base 2300 to be raised, so that the magnetic attraction members 2301 are brought to be close to the sample placement plate 2500, and the magnetic attraction members 2301 are thus capable of attracting magnetic particles with nucleic acids, and after that, the waste liquid in the sample placement hole 2501 is removed using the sample adding pump and the sample adding probe. Since the magnetic particles with nucleic acids are attracted by the magnetic attraction members 2301, they will not be removed by the sample adding probe, which in turn can realize the separation of the waste liquid from nucleic acids, thus realizing the extraction of nucleic acids. It is to be noted that the sample placement plate 2500 may be a deep-hole plate or a microplate.

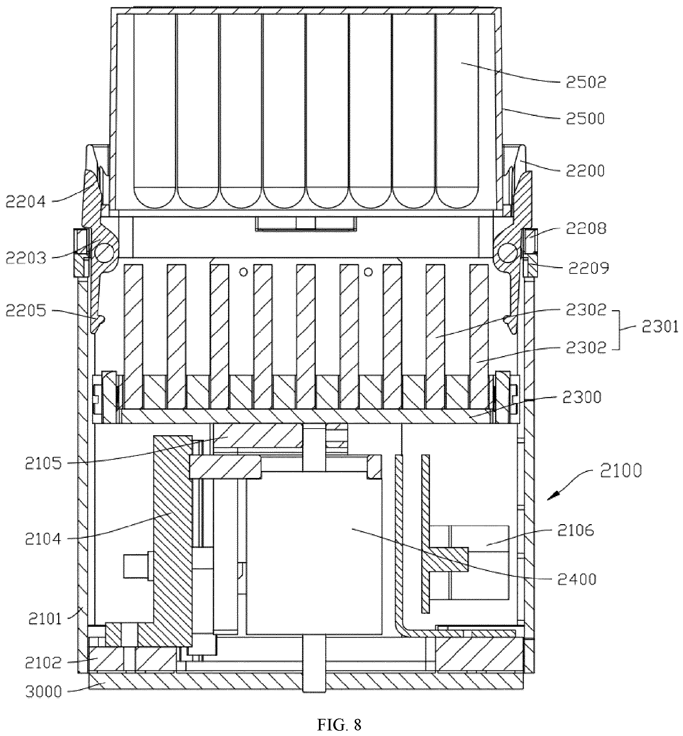

Referring to FIGS. 7 and 8, in some embodiments, the sample placement plate base 2200 is formed with a relief opening 2201 that is penetrated from top to bottom, wherein the relief opening 2201 is formed with a carrying platform 2202 on a side wall, the carrying platform 2202 is used to carry the sample placement plate 2500. When the sample placement plate 2500 needs to be placed, the sample placement plate 2500 is moved downwardly from above the sample placement plate base 2200, so that the sample placement plate 2500 is carried on the carrying platform 2202. When the sample placement plate 2500 needs to be taken out, the sample placement plate 2500 is taken out from the top end of the sample placement plate base 2200. Therefore, the structure is simple and easy to operate. Of course, the sample placement plate 2500 may be mounted in other ways. For example, the sample placement plate 2500 may be clamped and fixed by means of a fixture.

**In** some embodiments, as shown in FIGS. 6 to 8, the sample placement plate 2500 is provided with a plurality of sample placement tubes 2502 side by side, with the sample placement holes 2501 being formed within the sample placement tubes 2502, and the magnetic attraction members 2301 include a plurality of magnetic attraction rods 2302 disposed side by side on a top end of the magnetic attraction base 2300, wherein when the magnetic attraction base 2300 is at the first position, the magnetic attraction rods 2302 are inserted at the circumferential side of the sample placement tubes 2502. Specifically, the sample placement tubes 2502 are formed with avoidance space at the circumferential side, and the magnetic attraction rods 2302 are inserted in the avoidance space at the circumferential side of the sample placement tubes 2502 when the magnetic attraction base 2300 is at the first position, thereby facilitating the adsorption of magnetic particles with nucleic acids in the sample placement holes 2501 by the magnetic attraction rods 2302 and providing a good effect of attracting the magnetic particles with nucleic acids and thus a good effect of extracting nucleic acids.

It is to be noted that the magnetic attraction rods 2302 may be rectangular, or cylindrical, or in other suitable shapes. It can be understood that the magnetic attraction members 2301 may also include a plurality of magnetic attraction rings disposed side by side on the top end of the magnetic attraction base 2300. When the magnetic attraction base 2300 is at the first position, the magnetic attraction rings are arranged around the sample placement tubes 2502, thereby similarly facilitating the adsorption of magnetic particles with nucleic acids in the sample placement holes 2501, and providing a good effect of attracting the magnetic particles with nucleic acids and thus a good effect of extracting nucleic acids.

In some embodiments, the sample placement plate base 2200 is provided with a limiting mechanism, the limiting mechanism is configured for limiting upward movement of the sample placement plate 2500 during the raising of the magnetic attraction base 2300 to the first position. The limiting mechanism is provided, so that when the magnetic attraction base 2300 is raised to the first position, i.e., when the magnetic attraction rods 2302 are inserted at the circumferential side of the sample placement tubes 2502 or when the magnetic attraction rings are arranged around the sample placement tubes 2502, the limiting mechanism fastens the sample placement plate 2500, so as to prevent the sample placement plate 2500 from tipping over due to upward movement caused by pushing by the magnetic attraction rods 2302 or the magnetic attraction rings, thus enabling more stable mounting of the sample placement plate 2500.

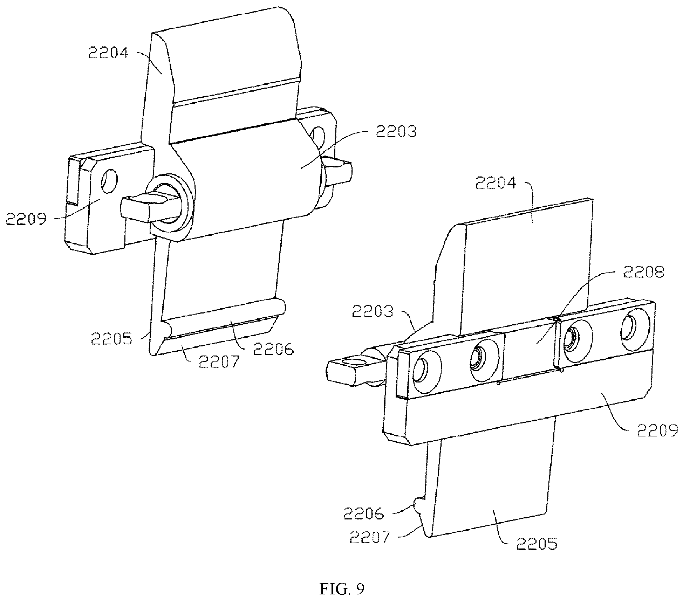

Referring to FIGS. 6 to 9, in some embodiments, the limiting mechanism includes clamping portions 2203 rotationally mounted on opposite sides of the sample placement plate base 2200, wherein the clamping portion 2203 is formed with a clamping end 2204 at the top end and a pushing end 2205 at the bottom end. During the process of raising the magnetic attraction base 2300 to the first position, the magnetic attraction base 2300 acts on the inner side wall of the pushing ends 2205 to push the pushing ends 2205 to move outwardly, which in turn causes the clamping ends 2204 to move inwardly to clamp the outer side wall of the sample placement plate 2500. Specifically, the axis of rotation of the clamping portion 2203 may be provided horizontally, and the axis of rotation of the clamping portion 2203 may be disposed at a position of the clamping portion 2203 that is close to the middle in the vertical direction, and after the sample placement plate 2500 is carried on the carrying platform 2202, the axis of rotation of the clamping portion 2203 may be parallel to the side wall of the sample placement plate 2500 close to the clamping portion 2203.

In this embodiment, in the process of the magnetic attraction base 2300 being raised to the first position, the magnetic attraction base 2300 gradually reaches between the pushing ends 2205 of the two clamping portions 2203, and the outer side of the magnetic attraction base 2300 abuts against the inner side walls of the pushing ends 2205 of the clamping portions 2203 and pushes the pushing ends 2205 of the clamping portions 2203 to move outwardly. When the pushing ends 2205 of the clamping portions 2203 are moved outwardly, the clamping portions 2203 can be rotated in a set direction, and the clamping ends 2204 of the clamping portions 2203 can be moved inwardly to clamp the outer side wall of the sample placement plate 2500, thereby limiting the upward movement of the sample placement plate 2500. When the extraction of nucleic acids is completed, the magnetic attraction base 2300 is moved downwardly to remove the magnetic attraction base 2300 from between the pushing ends 2205 of the two clamping portions 2203, so that the pushing ends 2205 of the clamping portions 2203 are no longer under the action of the pushing force of the magnetic attraction base 2300 and can thus be moved inwardly, then the clamping portions 2203 can be rotated in the direction opposite to the set direction, and the clamping ends 2204 of the clamping portions 2203 can be moved outwardly so as to release the clamping on the outer side wall of the sample placement plate 2500, thereby facilitating the removal of the sample placement plate 2500. Therefore, the structure is simple and easy to operate.

It is to be noted that the limiting mechanism may also be of other structures. For example, it may be a limiting pin slidably mounted on the sample placement plate base 2200, and the sample placement plate 2500 may be correspondingly provided with a limiting hole on the outer side wall. After the sample placement plate 2500 is carried on the carrying platform 2202, the limiting pin is inserted into the limiting hole, which is likewise capable of preventing upward movement of the sample placement plate 2500.

It can be understood that the inner sides of the pushing ends 2205 in the present invention refer to the sides of the two pushing ends 2205 that are close to each other, the pushing ends 2205 moving outwardly means that the two pushing ends 2205 move in a direction away from each other, the pushing ends 2205 moving inwardly means that the two pushing ends 2205 move in a direction approaching each other, the clamping ends 2204 moving outwardly means that the two clamping ends 2204 move in a direction away from each other, and the clamping end 2204 moving inwardly means that the two clamping ends 2204 move in a direction approaching each other.

In some embodiments, the pushing end 2205 is provided with a convex portion 2206 on the inner side wall, the convex portion 2206 extends along the axis of rotation of the clamping portion 2203 and the convex portion 2206 has a semi-circular cross-section in the length direction. With this arrangement, when the magnetic attraction base 2300 is moved upwardly to abut against the bottoms of the convex portions 2206 and continues to be moved upwardly, it can slide to the inner sides of the convex portions 2206, and can thus act on the convex portions 2206, thereby facilitating pushing the pushing ends 2205 to move outwardly. It can be understood that the inner sides of the convex portions 2206 in the present invention refer to the sides of the two convex portions 2206 that are close to each other.

In some embodiments, the inner side wall of the pushing end 2205 is formed with a first inclined face 2207 below the convex portion 2206, the first inclined face 2207 extends obliquely in a downward and outward direction. With this arrangement, the distance between the first inclined faces 2207 of the two pushing ends 2205 is gradually increased in the downward direction, thereby facilitating the reaching of the magnetic attraction base 2300 between the two pushing ends 2205, and when the magnetic attraction base 2300 continues to move upwardly, it can also push the pushing ends 2205 of the clamping portions 2203 to move outwardly for a certain distance, thereby facilitating the sliding of the magnetic attraction base 2300 to the inner sides of the convex portions 2206.

In some embodiments, a reset structure is provided between the sample placement plate base 2200 and the clamping portion 2203, the reset structure is capable of moving the clamping end 2204 to move outwardly. When the extraction of nucleic acids is completed, the magnetic attraction base 2300 is moved downwardly to remove the magnetic attraction base 2300 from between the pushing ends 2205 of the two clamping portions 2203, so that at this time, the pushing ends 2205 of the clamping portions 2203 are no longer under the action of the pushing force of the magnetic attraction base 2300, and under the action of the reset structures, it is possible to cause the clamping ends 2204 to move outwardly and cause the pushing ends 2205 to move inwardly so as to release the clamping on the outer side wall of the sample placement plate 2500, thereby facilitating the removal of the sample placement plate 2500. Therefore, the structure is simple and easy to operate.

In some embodiments, the reset structure includes an electromagnet 2208 provided at the sample placement plate base 2200, wherein the electromagnet 2208, when energized, attracts and engages the clamping end 2204 to cause the clamping end 2204 to move outwardly. Specifically, the sample placement plate base 2200 may be mounted with a mounting block 2209 by a fastener, and the electromagnet 2208 may be mounted on the mounting block 2209. When the extraction of nucleic acids is completed, the magnetic attraction base 2300 is moved downwardly to remove the magnetic attraction base 2300 from between the pushing ends 2205 of the two clamping portions 2203, so that at this time, the pushing ends 2205 of the clamping portions 2203 are no longer under the action of the pushing force of the magnetic attraction base 2300, and after that, the electromagnets 2208 are energized, and the electromagnets 2208 can attract and engage the clamping ends 2204 to cause the clamping ends 2204 to move outwardly, which can then release the clamping on the outer side wall of the sample placement plate 2500, thereby facilitating the removal of the sample placement plate 2500. Therefore, the structure is simple and easy to operate.

It is to be noted that the reset structure may also be of other structures. For example, the reset structure may be a tension spring provided between the sample placement plate base 2200 and the clamping portion 2203, and the clamping end 2204 may be moved outwardly by the elastic restoring force of the tension spring. Of course, when the clamping portion 2203 is mounted to the sample placement plate base 2200 by a mounting shaft, the reset structure may also be a torsion spring arranged around the mounting shaft, wherein the two ends of the torsion spring are connected to the sample placement plate base 2200 and the clamping portion 2203, respectively, and the elastic restoring force of the torsion spring can cause the clamping end 2204 to move outwardly.

In some embodiments, as shown in FIGS. 6 to 8, the mounting base 2100 may include a module shell 2101 that is penetrated from top to bottom, the module shell 2101 may be provided with a bottom plate 2102 at the bottom end, the sample placement plate base 2200 may be mounted at the top end of the module shell 2101, and the bottom plate 2102 may be provided with a support member 2103 within the module shell 2101, the support member 2103 is used to support the sample placement plate base 2200. The module shell 2101 may be provided with a mounting portion 2104 on the inner side wall, a connecting portion 2105 may be slidably mounted on the mounting portion 2104 in the vertical direction, and the magnetic attraction base 2300 may be mounted on the connecting portion 2105. The driving mechanism 2400 may be a linear motor disposed between the connecting portion 2105 and the bottom plate 2102, and upon activation of the linear motor, the linear motor may control the connecting portion 2105 to slide up and down, thereby realizing the raising and lowering of the magnetic attraction base 2300. Of course, the driving mechanism 2400 may also be of other structures. For example, it may be an air cylinder or an oil cylinder, as long as it is capable of controlling the raising and lowering of the magnetic attraction base 2300. In some embodiments, the module shell 2101 may also be provided with a magnetic attraction controller 2106, the magnetic attraction controller 2106 is used to control the operation of the linear motor. The mounting base 2100 may be mounted on a medical device via an adapter assembly 3000. It can be understood that the display and control assembly 1600 can control the movement of the linear motor by connecting to the magnetic attraction controller 2106, and the display and control assembly 1600 can also drive the linear motor directly by connecting to the linear motor.

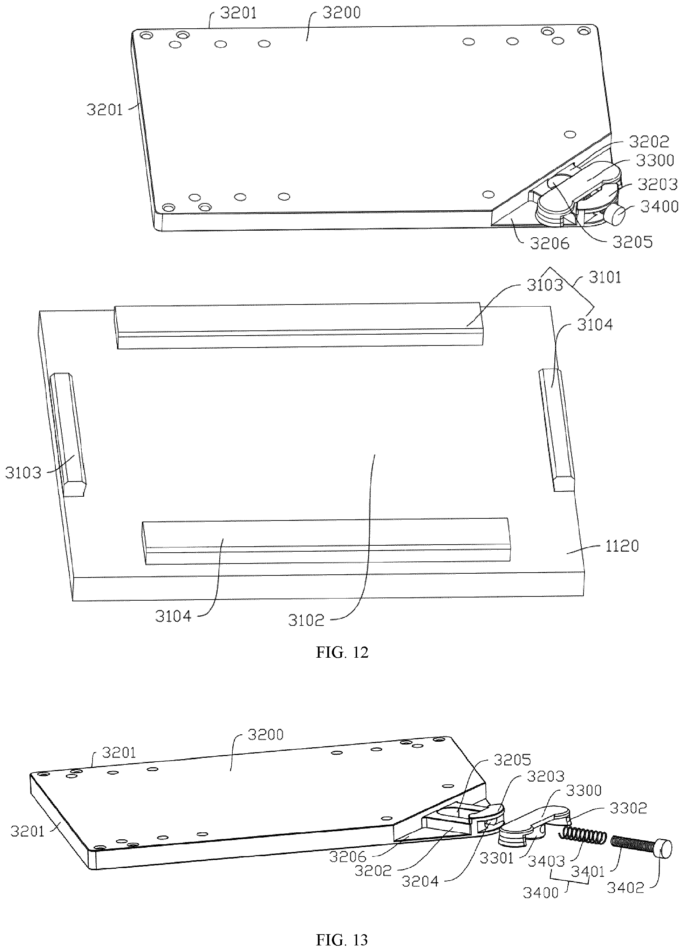

In some embodiments, referring to FIG. 1 and FIGS. 10 to FIG. 13, each of the plate positions 1130 is provided with a positioning structure 3101 on an upper end face thereof, and a mounting space 3102 is formed by the positioning structure 3101. The pipetting robot further includes an adapter assembly 3000, the adapter assembly 3000 is detachably provided within the mounting space 3102. At least one of the reagent unit, the sample unit, the magnetic attraction module, and the sample placement plate 2500 is provided on the plate positions 1130 by the adapter assembly 3000. The reagent unit, the sample unit, the magnetic attraction module, and the sample placement plate 2500, among others, can all be fixed to the adapter assembly 3000, so that they can be further fixed in the plate positions 1130 using the adapter assembly 3000. It can be understood that when fixing arrangement is not required, it is possible to not use an adapter module or to not fix the adapter module.

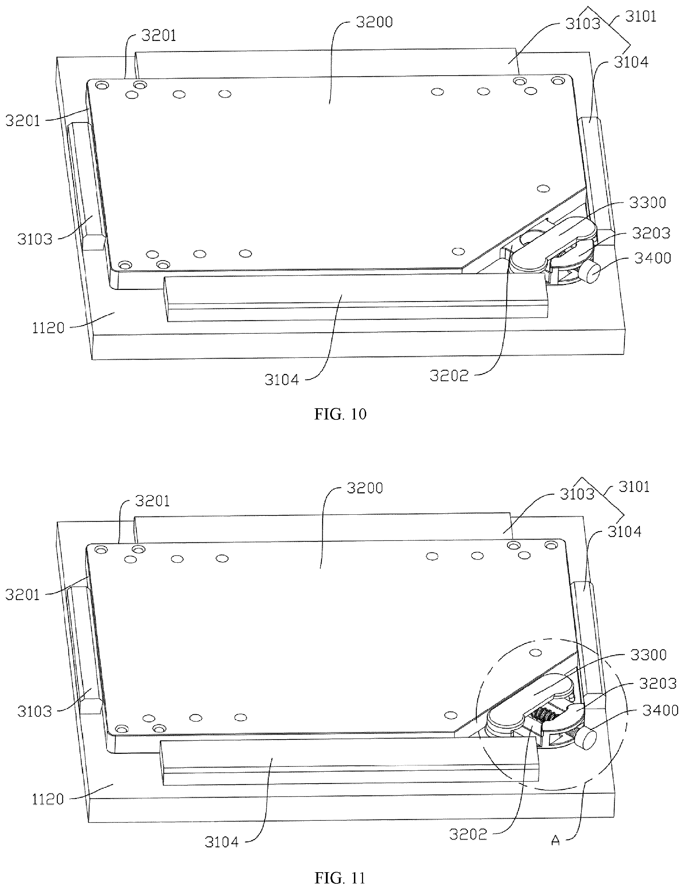

Referring to FIGS. 10 to 13, in some embodiments, the adapter assembly 3000 includes: an adapter base 3200, an abutting structure 3300, and a driving structure 3400, wherein:
the adapter base 3200 is arranged in the mounting space 3102, the positioning structure 3101 is located on a circumferential side of the adapter base 3200, and an outer side wall of the adapter base 3200 is formed with at least one abutting wall 3201;
the abutting structure 3300 is movably provided on the adapter base 3200, the abutting structure 3300 has a third position and a fourth position during movement, wherein, at the third position, the abutting structure 3300 abuts tightly against the positioning structure 3101 and causes the abutting wall 3201 to abut tightly against the positioning structure 3101 to realize mounting and fixing of the adapter base 3200, and at the fourth position, the abutting structure 3300 is detached from the positioning structure 3101 to release the mounting and fixing of the adapter base 3200; and
the driving structure 3400 is provided between the adapter base 3200 and the abutting structure 3300 to control movement of the abutting structure 3300.

**In** this embodiment, when the adapter base 3200 needs to be mounted, the adapter base 3200 is placed in the mounting space 3102, and then the driving structure 3400 is operated so that the driving structure 3400 controls the abutting structure 3300 to move to a third position in a direction approaching the positioning structure 3101, at which time the abutting structure 3300 abuts tightly against the corresponding position on the positioning structure 3101, and when the driving structure 3400 continues to act on the abutting structure 3300, since the abutting structure 3300 cannot continue to move due to the limitation by the positioning structure 3101, it can then react on the adapter base 3200 to cause a slight movement of the adapter base 3200, so that the abutting wall 3201 of the adapter base 3200 can abut tightly against the corresponding position on the positioning structure 3101, thereby realizing the mounting and fixing of the adapter base 3200. When the adapter base 3200 needs to be detached, the driving structure 3400 is operated so that the driving structure 3400 controls the abutting structure 3300 to move to the fourth position in a direction away from the positioning structure 3101, at which time the abutting structure 3300 is separated from the positioning structure 3101, and since the acting force exerted by the positioning structure 3101 on the abutting structure 3300 disappears, the abutting wall 3201 of the adapter base 3200 will not be abutted tightly against the positioning structure 3101, thereby releasing the mounting and fixing of the adapter base 3200, after which the adapter base 3200 is taken out of the mounting space 3102. Therefore, the operation is simple and convenient, thus saving time and labor.

It is to be noted that the positioning structure 3101 may include a plurality of positioning strips, the plurality of positioning strips forms the mounting space 3102 between them. Of course, the positioning structure 3101 may also be of an integral structure, with the inner side wall of the positioning structure 3101 forming the mounting space 3102 by enclosure. The adapter base 3200 may be of a plate-like structure, and the adapter base 3200 may be provided with mounting holes, the mounting holes are configured for mounting functional modules (e.g., a magnetic attraction module, an oscillation module, and the like), and the positions and numbers of the mounting holes may be different on different adapter bases 3200 so as to facilitate mounting of the corresponding functional modules. The adapter base 3200 may be formed with one or more abutting walls 3201 on the outer side wall, such as two abutting walls 3201 with an angle between them. The edge of the adapter base 3200 may be provided with an indentation 3206, and the abutting structure 3300 may be mounted on the indentation 3206 in a horizontally movable and adjustable manner. Of course, the direction of movement of the abutting structure 3300 may also be slightly tilted with respect to the horizontal direction, as long as it can be moved to abut tightly against the side of the positioning structure 3101 that is close to the adapter base 3200 or be separated from the positioning structure 3101. **In** addition, the abutting structure 3300 may also be mounted on the outer side of the adapter base 3200.

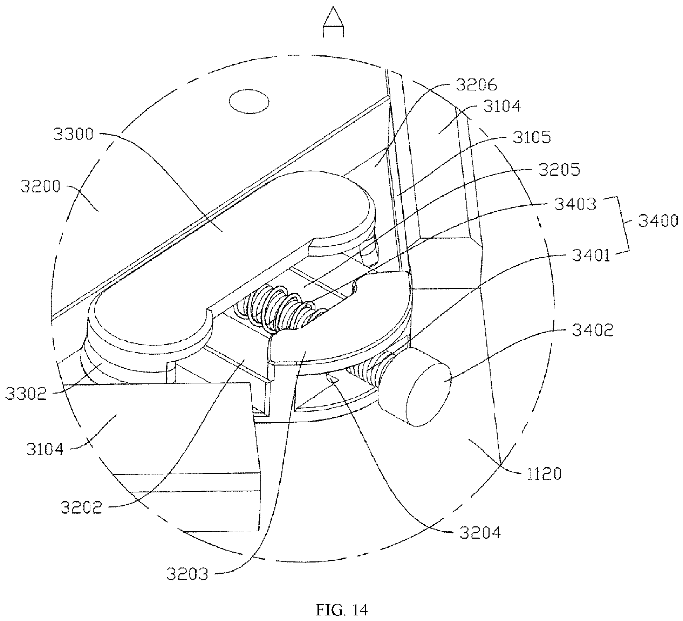

Referring to FIGS. 10 to 14, in some embodiments, the adapter base 3200 is provided with a guide portion 3202, the abutting structure 3300 is slidably mounted on the guide portion 3202, and the guide portion 3202 is provided with a limiting portion 3203 at a tail end, with the limiting portion 3203 being provided with an avoidance hole 3204, and the driving structure 3400 includes a driving screw 3401, wherein the driving screw 3401 passes through the avoidance hole 3204 and is threadedly connected to the abutting structure 3300, and the driving screw 3401 is provided with a screw head 3402, the screw head 3402 is located on a side of the limiting portion 3203 away from the abutting structure 3300, and the screw head 3402 is of a size greater than the size of the avoidance hole 3204. When the adapter base 3200 needs to be mounted and fixed, the screw head 3402 is made to abut against a side of the limiting portion 3203 away from the abutting structure 3300, and then the driving screw 3401 is rotated in a set direction to cause the screw head 3402 and the abutting structure 3300 to approach each other. Since the screw head 3402 abuts against the side of the limiting portion 3203 away from the abutting structure 3300, the screw head 3402 cannot be moved, so that the abutting structure 3300 can be caused to move in the direction approaching the positioning structure 3101. When the abutting structure 3300 is at the third position of abutting tightly against the positioning structure 3101, mounting and fixing of the adapter base 3200 can be realized. When the mounting and fixing of the adapter base 3200 needs to be released, it is only necessary to rotate the driving screw 3401 in the direction opposite to the set direction. Therefore, the structure is simple and easy to operate.

It is to be noted that when the edge of the adapter base 3200 is provided with the indentation 3206, the guide portion 3202 may be connected to the side wall of the indentation 3206, wherein the tail end of the guide portion 3202 refers to an end of the guide portion 3202 away from the side wall of the indentation 3206. In addition, the driving structure 3400 may also be of other structures. For example, it may be a driving oil cylinder which can likewise control the movement of the abutting structure 3300 through the stretching and contracting of the driving oil cylinder.

In some embodiments, the driving structure 3400 further includes a spring 3403 arranged around the driving screw 3401, two ends of the spring 3403 abuts against the sides of the abutting structure 3300 and the limiting portion 3203 that are close to each other, respectively. When the driving screw 3401 is rotated in the set direction to cause the screw head 3402 and the abutting structure 3300 to approach each other, the spring 3403 is compressed, whereas when the driving screw 3401 is rotated in the direction opposite to the set direction, the spring 3403 is gradually reset and elongated so that it can push the abutting structure 3300 to move towards the direction away from the positioning structure 3101, and thus enable quick resetting of the abutting structure 3300, thereby realizing more convenient use.

In some embodiments, the guide portion 3202 is provided with a sliding slot 3205 that is open at the top end, the abutting structure 3300 is provided with a sliding portion 3301, the sliding portion 3301 is slidably mounted in the sliding slot 3205, the avoidance hole 3204 is in communication with the sliding slot 3205, and the driving screw 3401 is threadedly connected to the sliding portion 3301. The abutting structure 3300 is slidably mounted on the guide portion 3202 through the cooperation of the sliding portion 3301 and the sliding slot 3205, and the driving screw 3401 is received in the sliding slot 3205 after passing through the avoidance hole 3204, which not only makes the overall structure of the adapter assembly 3000 more compact but also makes the exposed portion of the driving screw 3401 smaller, and thus being not prone to adhering dust or being damaged. It is to be noted that the abutting structure 3300 may also be slidably mounted on the guide portion 3202 in other manners. For example, a sliding rail may be provided on the guide portion 3202, with the abutting structure 3300 being slidably mounted on the sliding rail.

In some embodiments, in a horizontal direction perpendicular to the sliding direction of the abutting structure 3300, a first gap is reserved between the avoidance hole 3204 and the driving screw 3401, and the sliding portion 3301 is provided in a cylindrical shape, a centerline of the sliding portion 3301 extends in the vertical direction, and a second gap is reserved between the sliding portion 3301 and the inner side walls on the two sides of the sliding slot 3205. In this embodiment, with this arrangement, the abutting structure 3300 can be adjusted for fine movement along the directions of the two sides of the sliding slot 3205, and at the same time, the abutting structure 3300 can be adjusted for fine rotation around the centerline of the sliding portion 3301. Therefore, even if there is an error in the machining accuracy of the adapter base 3200, the positioning structure 3101 and the abutting structure 3300, by making fine adjustments to the abutting structure 3300, the abutting structure 3300 is made less prone to jamming during the movement of the abutting structure 3300, and at the same time, it is possible to enable the abutting structure 3300 to accurately abut against the corresponding position on the positioning structure 3101 and the abutting wall 3201 of the adapter base 3200 to accurately abut against the corresponding position of the positioning structure 3101, so as to provide a better mounting and fixing of the adapter base 3200.

It is to be noted that the avoidance hole 3204 may be a waist-shaped hole and the cross-section of the avoidance hole 3204 may extend in the horizontal direction perpendicular to the sliding direction of the abutting structure 3300. Of course, the avoidance hole 3204 may also be a round hole and have a diameter larger than the diameter of the driving screw 3401. Both arrangements of the avoidance hole 3204 can enable a first gap to be reserved between the avoidance hole 3204 and the driving screw 3401.

In some embodiments of the present invention, as shown in FIGS. 10 to 14, the end face of the limiting portion 3203 facing the screw head 3402 is provided as an outwardly convex arc surface, and the arc centerline of the end face of the limiting portion 3203 facing the screw head 3402 extends in the vertical direction. With this arrangement, even when the abutting structure 3300 is subjected to subtle movement along the directions of the two sides of the sliding slot 3205, or the abutting structure 3300 is subjected to subtle rotation around the centerline of the sliding portion 3301 to cause deflection of the driving screw 3401, the screw head 3402 can conveniently abut against the end face of the limiting portion 3203 facing the screw head 3402, thereby having a better control on the movement of the abutting structure 3300.

In some embodiments, the adapter base 3200 has two abutting walls 3201, the two abutting walls 3201 adjoins each other and has an angle therebetween, and the place where the two abutting walls 3201 adjoin and the abutting structure 3300 are diagonally distributed on the adapter base 3200. Specifically, the angle between the two abutting walls 3201 may be 90 degrees, and the abutting structure 3300 may move approximately along a diagonal direction of the adapter base 3200. In this embodiment, two abutting walls 3201 are provided, and when the abutting structure 3300 is at the third position, both abutting walls 3201 abut tightly against the positioning structure 3101, and since there is an angle between the two abutting walls 3201 and the places where the abutting structure 3300 adjoins the two abutting walls 3201 are diagonally distributed on the adapter base 3200, it is possible to make the adapter base 3200 abut tightly against the positioning structure 3101 at a plurality of positions and along a plurality of directions, so that it is possible to avoid the movement of the adapter base 3200 along the horizontal direction, thus enabling more stable mounting and fixing of the adapter base 3200. In addition, by moving and adjusting the abutting structure 3300, the two abutting walls 3201 can be made to synchronously abut tightly against the corresponding positions on the positioning structure 3101 or to be released from the abutting state with the corresponding positions on the positioning structure 3101, and thus the operation is more convenient.

It is to be noted that the angle between the two abutting walls 3201 may also have other angular values, such as 60 degrees or 120 degrees. The number of abutting walls 3201 may also be other values, such as one or three. When there is one abutting wall 3201, the abutting wall 3201 and the abutting structure 3300 may be located at the two ends of the adapter base 3200, respectively, and the abutting wall 3201 may be an outwardly convex arc surface, which is in turn able to likewise avoid the movement of the adapter base 3200 in the horizontal direction. When there are three abutting walls 3201, the three abutting walls 3201 may adjoin one another in sequence, and the angle between two adjacent abutting walls 3201 may be 120 degrees.

In some embodiments, the positioning structure 3101 is provided with a first positioning strip 3103 corresponding to each abutting wall 3201, wherein the first positioning strip 3103 is disposed on the outer side of the corresponding abutting wall 3201 and extends along the length direction of the corresponding abutting wall 3201, and when the abutting structure 3300 is at the first position, the abutting wall 3201 abuts tightly against the corresponding first positioning strip 3103. This arrangement makes the abutting wall 3201 more adapted to the positioning structure 3101, and the abutting wall 3201 can better abut tightly against the positioning structure 3101, thereby providing better mounting and fixing for the adapter base 3200.

In some embodiments, the positioning structure 3101 is provided with a second positioning strip 3104 corresponding in parallel to each of the first positioning strips 3103, and the abutting structure 3300 is provided with abutting portions 3302 on both sides of the abutting structure 3300 in the movement direction. When the abutting structure 3300 is at the first position, the two abutting portions 3302 abut tightly against the two second positioning strips 3104, respectively. This arrangement enables the adapter base 3200 to abut tightly against the positioning structure 3101 at more positions and in more directions, so that it is more capable of avoiding movement of the adapter base 3200 in the horizontal direction, thus enabling more stable mounting and fixing of the adapter base 3200. Moreover, the abutting structure 3300 abuts tightly against the two second positioning strips 3104 through the two abutting portions 3302, which makes it possible to avoid the phenomenon of stress concentration, thus avoiding damages to the abutting structure 3300. In addition, since the second positioning strips 3104 correspond to the first positioning strips 3103 in parallel, when the abutting portions 3302 abut tightly against the second positioning strips 3104 so that the second positioning strips 3104 react on the adapter base 3200, it is possible to enable the two abutting walls 3201 to accurately abut against the two first positioning strips 3103, thus enabling the adapter base 3200 to be mounted and fixed in a more rapid and accurate manner.

In some embodiments, the side wall of the second positioning strip 3104 facing the adapter base 3200 has a second inclined face 3105, the second inclined face 3105 extends upward and in a direction approaching the adapter base 3200. When the abutting structure 3300 is at the first position, the abutting portion 3302 abuts tightly against the second inclined face 3105 and/or the side wall of the second positioning strip 3104 that is below the second inclined face 3105. The second inclined faces 3105 are provided, and the second inclined faces 3105 are capable of limiting the abutting portions 3302 so as to avoid upward movement of the abutting portions 3302, which makes it possible to limit upward displacement of the adapter base 3200, thus enabling more secure mounting and fixing of the adapter base 3200.

It is to be noted that when the abutting structure 3300 is at the third position, the abutting portions 3302 may be abutted tightly against the second inclined faces 3105 and may also be abutted tightly against the side walls of the second positioning strips 3104 that are below the second inclined faces 3105, and may also be partially abutted tightly against the second inclined faces 3105 and partially abutted tightly against the side walls of the second positioning strips 3104 that are below the second inclined faces 3105.

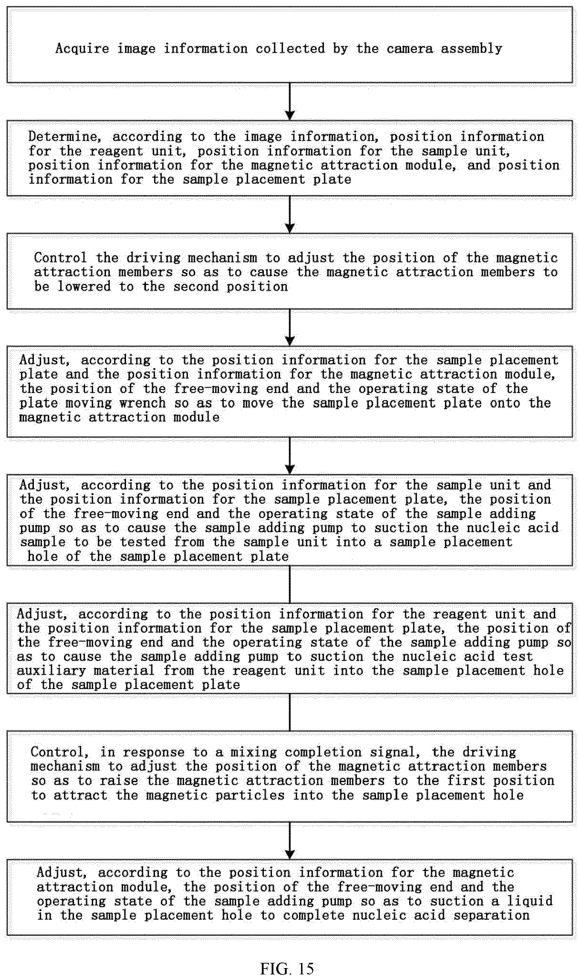

Referring to FIG. 15, which is a flowchart of a pipetting robot control method as provided in an embodiment of the present invention, the pipetting robot control method includs the following steps:
acquiring image information collected by the camera assembly 1500;
determining, according to the image information, position information for the reagent unit, position information for the sample unit, position information for the magnetic attraction module, and position information for the sample placement plate 2500;
controlling the driving mechanism 2400 to adjust the position of the magnetic attraction members 2301 so as to cause the magnetic attraction members 2301 to be lowered to the second position;
adjusting, according to the position information for the sample placement plate 2500 and the position information for the magnetic attraction module, the position of the free-moving end and the operating state of the plate moving wrench 1410 so as to move the sample placement plate 2500 onto the magnetic attraction module;
adjusting, according to the position information for the sample unit and the position information for the sample placement plate 2500, the position of the free-moving end and the operating state of the sample adding pump so as to cause the sample adding pump to suction the nucleic acid sample to be tested from the sample unit into a sample placement hole 2501 of the sample placement plate 2500;
adjusting, according to the position information for the reagent unit and the position information for the sample placement plate 2500, the position of the free-moving end and the operating state of the sample adding pump so as to cause the sample adding pump to suction the nucleic acid test auxiliary material from the reagent unit into the sample placement hole 2501 of the sample placement plate 2500;
controlling, in response to a mixing completion signal, the driving mechanism 2400 to adjust the position of the magnetic attraction members 2301 so as to raise the magnetic attraction members 2301 to the first position so that the magnetic particles are attracted into the sample placement hole 2501; and
adjusting, according to the position information for the magnetic attraction module, the position of the free-moving end and the operating state of the sample adding pump so as to suction a liquid in the sample placement hole 2501 to complete nucleic acid separation.

The specific structure of the pipetting robot has been described in more detail in the foregoing embodiments and will not be repeated here. **In** this embodiment, the description of the control method is completed based on the pipetting robot architecture described above. After the pre-work has been conducted, a working component currently connected to the free-moving end is determined through the display and control assembly 1600, and a nucleic acid separation strategy is selected. Afterwards, the nucleic acid separation can be automatically performed by the pipetting robot. The process of the nucleic acid separation strategy is as follows.

First, image information for the interior of the pipetting robot is continuously acquired by the camera assembly 1500, and the image information can be used to determine the position information for the reagent unit, the position information for the sample unit, the position information for the magnetic attraction module, the position information for the sample placement plate 2500, the position information for the sample adding probe box, and the position information for the waste probe box. At the same time, it can facilitate continuous observation of the interior of the sealing shell by the tester, so that timely manual intervention can be made in the event of problems inside the sealing shell to avoid expansion of the situation.

In order to ensure that the nucleic acid separation test is performed properly, it is necessary to lower the magnetic attraction members 2301 to the second position prior to sample adding to ensure that the magnetic attraction members 2301 do not affect the test material in the magnetic attraction module after the sample placement plate 2500 has been moved onto the magnetic attraction module.

After the magnetic attraction members 2301 are adjusted to the first position, the multi-directional movement assembly 1300 can be adjusted to use the plate moving wrench 1410 to clamp the sample placement plate 2500 and move it to the magnetic attraction module. During this process, because there is no liquid in the sample placement plate 2500, a high moving speed can be maintained. Thereafter, the multi-directional movement assembly 1300 is adjusted to move the sample adding pump to the region where the sample adding probe box is located to suction a sample adding probe, and control the sample adding pump to the sample unit to suction a nucleic acid sample to be tested into a sample placement hole 2501 of the sample placement plate 2500, followed by discarding the sample adding probe into the waste probe box. It can be understood that the sample adding probe needs to be replaced once after each liquid suctioning operation to avoid contamination of the sample, and the subsequent process of suctioning the sample adding probe and discarding the sample adding probe will not be further described.

After adding the nucleic acid sample to be tested, the sample adding pump is then used to suction the nucleic acid test auxiliary material from the reagent unit into the sample placement hole 2501 of the sample placement plate 2500 to enable the nucleic acid test auxiliary material to be mixed with the nucleic acid sample to be tested and to enable magnetic particles in the nucleic acid test auxiliary material to complete adsorption of nucleic acids.

Once the adsorption is completed, the magnetic attraction members 2301 can be controlled to be raised so as to attract the magnetic particles in the sample placement hole 2501, after which the sample adding pump can be used to suction the liquid out of the sample placement hole 2501, and the nucleic acids, because of being adsorbed by the magnetic particles, are left behind in the sample placement hole 2501, thereby realizing the separation of the nucleic acids. The sample placement plate 2500, from which the waste liquid has been suctioned, can likewise be moved quickly after being clamped, without the need for careful and slow movement as in the case where liquids are present.

**In** some embodiments, the pipetting robot further includes a lid opening grasper 1420 electrically connected to the display and control assembly 1600. The sample adding pump includes an inline sample adding pump 1430 and a single probe sample adding pump 1440; and the display and control assembly 1600 is preset with a plurality of test operation strategies, wherein each of the test operation strategies corresponds to one combined test operation mechanism, the combined test operation mechanism includes at least a combination of the inline sample adding pump 1430 with the plate moving wrench 1410, a combination of the single probe sample adding pump 1440 with the plate moving wrench 1410, a combination of the inline sample adding pump 1430 with the lid opening grasper 1420, a combination of the single probe sample adding pump 1440 with the lid opening grasper 1420, and a combination of the inline sample adding pump 1430 with the single probe sample adding pump 1440, and each of the test operation strategies is configured for controlling a corresponding combined test operation mechanism among the combined test operation mechanisms to complete a test operation, and the display and control assembly 1600 is configured for selecting a corresponding one of the test operation strategies according to the combined test operation mechanism.

The pipetting robot control method further includes the following steps:
acquiring combination information corresponding to a combined test operation mechanism connected to the free-moving end;
selecting a corresponding test operation strategy according to the combination information; and
executing the selected test operation strategy in response to an execution instruction from the user, wherein the test operation strategy includes at least a nucleic acid separation strategy.

Specifically, after the user has mounted the combined test operation mechanism, the display and control assembly 1600 can provide the test operation strategies that the current combined test operation mechanism can realize. Since in most cases, multiple tests can be realized by the same combined test operation mechanism, it is necessary for the user to select according to the displayed test operation strategies the test operation strategy that needs to be executed, and after the user determines the test operation strategy to be executed, the execution starts.

In addition, an embodiment of the present invention provides a computer-readable storage medium storing computer-executable instructions, wherein the computer-executable instructions, when executed by a processor or a control unit, can cause the above-mentioned processor to execute the pipetting robot control method in the embodiments of the present invention described above, for example, to execute the method described above.

Although the embodiments of the present invention have been described in detail above with reference to the accompanying drawings, the present invention is not limited to the above embodiments, and various changes may be made within the knowledge of those of ordinary skill in the art without departing from the scope of the appended claims.

## Claims

1. A pipetting robot, **characterized in** comprising:
a sealing body (1100) comprising a mounting frame (1110) and a sealing shell wrapped around the mounting frame (1110), wherein the mounting frame (1110) is provided with a placement platform (1120) at a bottom of the mounting frame (1110), the placement platform (1120) is provided with a plurality of plate positions (1130), the plurality of the plate positions (1130) are configured for at least placing a reagent unit, a sample unit, a magnetic attraction module, and a sample placement plate (2500); wherein the sample unit is configured for placing a nucleic acid sample to be tested, and the reagent unit is configured for placing a nucleic acid test auxiliary material, the nucleic acid test auxiliary material comprises magnetic particles which are configured to mix with the nucleic acid sample to be tested; the sample placement plate (2500) has a plurality of sample placement holes (2501); and the magnetic attraction module comprises a driving mechanism (2400) and a plurality of liftable magnetic attraction members (2301) driven by the driving mechanism (2400), and the magnetic attraction module is configured for placing the sample placement plate (2500) and is capable of raising the magnetic attraction members (2301) to a first position close to the sample placement plate (2500) and lowering the magnetic attraction members (2301) to a second position away from the sample placement plate (2500);
an air filtration assembly (1200) provided on the sealing shell and configured for filtering and cleaning air entering and leaving the sealing shell;
a multi-directional movement assembly (1300) provided on the mounting frame (1110), the multi-directional movement assembly (1300) having a free-moving end, wherein the free-moving end is capable of performing multi-directional movement above the placement platform (1120);
a sample adding pump provided on the free-moving end, the sample adding pump being configured for injecting and suctioning a liquid;
a plate moving wrench (1410) provided on the free-moving end, the plate moving wrench (1410) being configured for moving the sample placement plate (2500);
a camera assembly (1500) provided within the sealing shell and configured for collecting image information for an interior of the sealing shell; and
a display and control assembly (1600) electrically connected separately to the driving mechanism (2400), the air filtration assembly (1200), the multi-directional movement assembly (1300), the sample adding pump, the plate moving wrench (1410), and the camera assembly (1500),
wherein each of the plate positions (1130) is provided with a positioning structure (3101) on an upper end face of the plate position (1130), and a mounting space (3102) is formed by the positioning structure (3101); the pipetting robot further comprises an adapter assembly (3000), the adapter assembly (3000) is detachably provided within the mounting space (3102); and at least one of the reagent unit, the sample unit, the magnetic attraction module, and the sample placement plate (2500) is provided on the plate position (1130) by the adapter assembly (3000); and
the adapter assembly (3000) comprises:
an adapter base (3200) arranged in the mounting space (3102), the positioning structure (3101) being located on a circumferential side of the adapter base (3200), and an outer side wall of the adapter base (3200) being formed with at least one abutting wall (3201);
an abutting structure (3300) movably provided on the adapter base (3200), during movement, the abutting structure (3300) having a third position where the abutting structure (3300) abuts tightly against the positioning structure (3101) and causes the abutting wall (3201) to abut tightly against the positioning structure (3101) to realize mounting and fixing of the adapter base (3200), and a fourth position where the abutting structure (3300) is detached from the positioning structure (3101) to release the mounting and fixing of the adapter base (3200); and
a driving structure (3400) provided between the adapter base (3200) and the abutting structure (3300) to control movement of the abutting structure (3300);
wherein the adapter base (3200) is provided with a guide portion (3202), the abutting structure (3300) is slidably mounted on the guide portion (3202), and the guide portion (3202) is provided with a limiting portion (3203) at a tail end, with the limiting portion (3203) being provided with an avoidance hole (3204), and the driving structure (3400) comprises a driving screw (3401), wherein the driving screw (3401) passes through the avoidance hole (3204) and is threadedly connected to the abutting structure (3300), and the driving screw (3401) is provided with a screw head (3402), the screw head (3402) is located on a side of the limiting portion (3203) away from the abutting structure (3300), and the screw head (3402) is of a size greater than a size of the avoidance hole (3204).

2. The pipetting robot according to claim 1, wherein the pipetting robot further comprises a lid opening grasper (1420) electrically connected to the display and control assembly (1600); the sample adding pump comprises an inline sample adding pump (1430) and a single probe sample adding pump (1440); and the display and control assembly (1600) is preset with a plurality of test operation strategies, wherein each of the test operation strategies corresponds to one combined test operation mechanism, the combined test operation mechanism comprises at least a combination of the inline sample adding pump (1430) with the plate moving wrench (1410), a combination of the single probe sample adding pump (1440) with the plate moving wrench (1410), a combination of the inline sample adding pump (1430) with the lid opening grasper (1420), a combination of the single probe sample adding pump (1440) with the lid opening grasper (1420), and a combination of the inline sample adding pump (1430) with the single probe sample adding pump (1440), and each of the test operation strategies is configured for controlling a corresponding combined test operation mechanism among the combined test operation mechanisms to complete a test operation, and the display and control assembly (1600) is configured for selecting a corresponding one of the test operation strategies according to the combined test operation mechanism.

3. The pipetting robot according to claim 1, wherein the magnetic attraction module comprises:
a mounting base (2100);
a sample placement plate base (2200) provided on the mounting base (2100), the sample placement plate base (2200) being configured for mounting the sample placement plate (2500);
a magnetic attraction base (2300) liftably mounted to the mounting base (2100) and located below the sample placement plate base (2200), the magnetic attraction base (2300) being provided with the magnetic attraction members (2301), the magnetic attraction base (2300) being capable of being raised to the first position where the magnetic attraction members (2301) are close to the sample placement plate (2500) and being lowered to the second position where the magnetic attraction members (2301) are away from the sample placement plate (2500); and
the driving mechanism (2400) provided on the mounting base (2100) and in transmission connection with the magnetic attraction base (2300), the driving structure (3400) being electrically connected to the display and control assembly (1600) for controlling the raising and lowering of the magnetic attraction base (2300).

4. The pipetting robot according to claim 3, wherein the sample placement plate (2500) is provided with a plurality of sample placement tubes (2502) side by side, with the sample placement holes (2501) being formed within the sample placement tubes (2502) respectively, and the magnetic attraction members (2301) comprise a plurality of magnetic attraction rods (2302) or magnetic attraction rings disposed side by side on a top end of the magnetic attraction base (2300), wherein when the magnetic attraction base (2300) is at the first position, each of the magnetic attraction rods (2302) is inserted at a circumferential side of a respective one of the sample placement tubes (2502), or each of the magnetic attraction rings is arranged around a respective one of the sample placement tubes (2502).

5. The pipetting robot according to claim 3, wherein the sample placement plate base (2200) is provided with a limiting mechanism, the limiting mechanism is configured for limiting upward movement of the sample placement plate (2500) during the raising of the magnetic attraction base (2300) to the first position.

6. A pipetting robot control method using the pipetting robot according to any one of claims 1 to 5, the pipetting robot control method comprising:
acquiring image information collected by the camera assembly (1500);
determining, according to the image information, position information for the reagent unit, position information for the sample unit, position information for the magnetic attraction module, and position information for the sample placement plate (2500);
controlling the driving mechanism (2400) to adjust the position of the magnetic attraction members (2301) so as to cause the magnetic attraction members (2301) to be lowered to the second position;
adjusting, according to the position information for the sample placement plate (2500) and the position information for the magnetic attraction module, a position of the free-moving end and an operating state of the plate moving wrench (1410) so as to move the sample placement plate (2500) onto the magnetic attraction module;
adjusting, according to the position information for the sample unit and the position information for the sample placement plate (2500), the position of the free-moving end and the operating state of the sample adding pump so as to cause the sample adding pump to suction the nucleic acid sample to be tested from the sample unit into the sample placement hole (2501) of the sample placement plate (2500);
adjusting, according to the position information for the reagent unit and the position information for the sample placement plate (2500), the position of the free-moving end and the operating state of the sample adding pump so as to cause the sample adding pump to suction the nucleic acid test auxiliary material from the reagent unit into the sample placement hole (2501) of the sample placement plate (2500), wherein the nucleic acid test auxiliary material comprises magnetic particles, and the magnetic particles mix with the nucleic acid sample to be tested in the sample placement hole (2501) of the sample placement plate (2500);
controlling, in response to a mixing completion signal, the driving mechanism (2400) to adjust the position of the magnetic attraction members (2301) so as to raise the magnetic attraction members (2301) to the first position to attract the magnetic particles into the sample placement hole (2501); and
adjusting, according to the position information for the magnetic attraction module, the position of the free-moving end and the operating state of the sample adding pump so as to suction a liquid in the sample placement hole (2501) to complete nucleic acid separation.

## Patentansprüche

1. Pipettierroboter, **dadurch gekennzeichnet, dass** er umfasst:
einen Dichtungskörper (1100), umfassend einen Montagerahmen (1110) und ein den Montagerahmen (1110) umhüllendes Dichtungsgehäuse, wobei der Montagerahmen (1110) mit einer Platzierungsplattform (1120) an einer Unterseite des Montagerahmens (1110) bereitgestellt ist, wobei die Platzierungsplattform (1120) mit einer Vielzahl von Plattenpositionen (1130) bereitgestellt ist, wobei die Vielzahl der Plattenpositionen (1130) zumindest zum Platzieren einer Reagenzieneinheit, einer Probeneinheit, eines magnetischen Anziehungsmoduls und einer Probenplatzierungsplatte (2500) konfiguriert sind; wobei die Probeneinheit zum Platzieren einer zu testenden Nukleinsäureprobe konfiguriert ist und die Reagenzieneinheit zum Platzieren eines Nukleinsäuretesthilfsmaterials konfiguriert ist, wobei das Nukleinsäuretesthilfsmaterial magnetische Partikel umfasst, die konfiguriert sind, um sich mit der zu testenden Nukleinsäureprobe zu mischen; wobei die Probenplatzierungsplatte (2500) eine Vielzahl von Probenplatzierungslöchern (2501) aufweist; und das magnetische Anziehungsmodul einen Antriebsmechanismus (2400) und eine Vielzahl von anhebbaren magnetischen Anziehungselementen (2301) umfasst, die durch den Antriebsmechanismus (2400) angetrieben werden, und das magnetischen Anziehungsmodul zum Platzieren der Probenplatzierungsplatte (2500) konfiguriert ist und in der Lage ist, die magnetischen Anziehungselemente (2301) in eine erste Position nahe der Probenplatzierungsplatte (2500) anzuheben und die magnetischen Anziehungselemente (2301) in eine zweite Position weg von der Probenplatzierungsplatte (2500) abzusenken;
eine Luftfilterbaugruppe (1200), die am Dichtungsgehäuse bereitgestellt und konfiguriert ist, Luft, die in das Dichtungsgehäuse eintritt und dieses verlässt, zu filtern und zu reinigen;
eine multidirektionale Bewegungsbaugruppe (1300), die an dem Montagerahmen (1110) bereitgestellt ist, wobei die multidirektionale Bewegungsbaugruppe (1300) ein frei bewegliches Ende aufweist, wobei das frei bewegliche Ende in der Lage ist, eine multidirektionale Bewegung über der Platzierungsplattform (1120) durchzuführen;
eine Probenzugabepumpe, die an dem sich frei bewegenden Ende bereitgestellt ist, wobei die Probenzugabepumpe zum Injizieren und Ansaugen einer Flüssigkeit konfiguriert ist;
einen Plattenbewegungsschlüssel (1410), der an dem frei beweglichen Ende bereitgestellt ist, wobei der Plattenbewegungsschlüssel (1410) zum Bewegen der Probenplatzierungsplatte (2500) konfiguriert ist;
eine Kamerabaugruppe (1500), die innerhalb des Dichtungsgehäuses bereitgestellt und konfiguriert ist, Bildinformationen für ein Inneres des Dichtungsgehäuses zu sammeln; und
eine Anzeige- und Steuerungsbaugruppe (1600), die elektrisch getrennt mit dem Antriebsmechanismus (2400), der Luftfilterbaugruppe (1200), der multidirektionalen Bewegungsbaugruppe (1300), der Probenzugabepumpe, dem Plattenbewegungsschlüssel (1410) und der Kamerabaugruppe (1500) verbunden ist,
wobei jede der Plattenpositionen (1130) mit einer Positionierungsstruktur (3101) an einer oberen Endfläche der Plattenposition (1130) bereitgestellt ist und ein Montageraum (3102) durch die Positionierungsstruktur (3101) ausgebildet ist; wobei der Pipettierroboter ferner eine Adapterbaugruppe (3000) umfasst, wobei die Adapterbaugruppe (3000) lösbar innerhalb des Montageraums (3102) bereitgestellt ist; und zumindest eine von der Reagenzieneinheit, der Probeneinheit, dem magnetischen Anziehungsmodul und der Probenplatzierungsplatte (2500) durch die Adapterbaugruppe (3000) an der Plattenposition (1130) bereitgestellt ist; und
die Adapterbaugruppe (3000) Folgendes umfasst:
eine Adapterbasis (3200), die in dem Montageraum (3102) eingerichtet ist, wobei sich die Positionierungsstruktur (3101) auf einer Umfangsseite der Adapterbasis (3200) befindet und eine äußere Seitenwand der Adapterbasis (3200) mit zumindest einer Anschlagwand (3201) ausgebildet ist;
eine Anschlagstruktur (3300), die während der Bewegung beweglich an der Adapterbasis (3200) bereitgestellt ist, wobei die Anschlagstruktur (3300) eine dritte Position, in der die Anschlagstruktur (3300) fest an der Positionierungsstruktur (3101) anliegt und bewirkt, dass die Anschlagwand (3201) fest an der Positionierungsstruktur (3101) anliegt, um die Montage und Befestigung der Adapterbasis (3200) auszuführen, und eine vierte Position aufweist, in der die Anschlagstruktur (3300) von der Positionierungsstruktur (3101) gelöst ist, um die Montage und Befestigung der Adapterbasis (3200) zu lösen; und
eine Antriebsstruktur (3400), die zwischen der Adapterbasis (3200) und der Anschlagstruktur (3300) bereitgestellt ist, um eine Bewegung der Anschlagstruktur (3300) zu steuern;
wobei die Adapterbasis (3200) mit einem Führungsabschnitt (3202) bereitgestellt ist, die Anschlagstruktur (3300) verschiebbar an dem Führungsabschnitt (3202) montiert ist und der Führungsabschnitt (3202) mit einem Begrenzungsabschnitt (3203) an einem hinteren Ende bereitgestellt ist, wobei der Begrenzungsabschnitt (3203) mit einem Vermeidungsloch (3204) bereitgestellt ist, und die Antriebsstruktur (3400) eine Antriebsschraube (3401) umfasst, wobei die Antriebsschraube (3401) durch das Vermeidungsloch (3204) verläuft und mit der Anschlagsstruktur (3300) verschraubt verbunden ist und die Antriebsschraube (3401) mit einem Schraubenkopf (3402) bereitgestellt ist, wobei sich der Schraubenkopf (3402) an einer Seite des Begrenzungsabschnitts (3203) entfernt von der Anschlagstruktur (3300) befindet und der Schraubenkopf (3402) eine Größe hat, die größer als eine Größe des Vermeidungslochs (3204) ist.

2. Pipettierroboter gemäß Anspruch 1, wobei der Pipettierroboter ferner einen Deckelöffnungsgreifer (1420) umfasst, der elektrisch mit der Anzeige- und Steuerungsbaugruppe (1600) verbunden ist; wobei die Probenzugabepumpe eine Inline-Probenzugabepumpe (1430) und eine Einzeluntersuchungsprobenzugabepumpe (1440) umfasst; und die Anzeige- und Steuerungsbaugruppe (1600) mit einer Vielzahl von Testbetriebsstrategien voreingestellt ist, wobei jede der Testbetriebsstrategien einem kombinierten Testbetriebsmechanismus entspricht, wobei der kombinierte Testbetriebsmechanismus zumindest eine Kombination der Inline-Probenzugabepumpe (1430) mit dem Plattenbewegungsschlüssel (1410), eine Kombination der Einzeluntersuchungsprobenzugabepumpe (1440) mit dem Plattenbewegungsschlüssel (1410), eine Kombination der Inline-Probenzugabepumpe (1430) mit dem Deckelöffnungsgreifer (1420), eine Kombination der Einzeluntersuchungsprobenzugabepumpe (1440) mit dem Deckelöffnungsgreifer (1420) und eine Kombination der Inline-Probenzugabepumpe (1430) mit der Einzeluntersuchungsprobenzugabepumpe (1440) umfasst, und jede der Testbetriebsstrategien zum Steuern eines entsprechenden kombinierten Testbetriebsmechanismus unter den kombinierten Testbetriebsmechanismen konfiguriert ist, um einen Testbetrieb abzuschließen, und die Anzeige- und Steuerungsbaugruppe (1600) zum Auswählen einer entsprechenden der Testbetriebsstrategien gemäß dem kombinierten Testbetriebsmechanismus konfiguriert ist.

3. Pipettierroboter gemäß Anspruch 1, wobei das magnetische Anziehungsmodul Folgendes umfasst:
eine Montagebasis (2100);
eine Probenplatzierungsplattenbasis (2200), die an der Montagebasis (2100) bereitgestellt ist, wobei die Probenplatzierungsplattenbasis (2200) zum Montieren der Probenplatzierungsplatte (2500) konfiguriert ist;
eine magnetische Anziehungsbasis (2300), die an der Montagebasis (2100) anhebbar montiert ist und sich unter der Probenplatzierungsplattenbasis (2200) befindet, wobei die magnetische Anziehungsbasis (2300) mit den magnetischen Anziehungselementen (2301) bereitgestellt ist, wobei die magnetische Anziehungsbasis (2300) in der Lage ist, in die erste Position angehoben zu werden, in der die magnetischen Anziehungselemente (2301) in der Nähe der Probenplatzierungsplatte (2500) sind, und in die zweite Position abgesenkt zu werden, in der die magnetischen Anziehungselemente (2301) von der Probenplatzierungsplatte (2500) entfernt sind; und
wobei der Antriebsmechanismus (2400) an der Montagebasis (2100) bereitgestellt ist und in Übertragungsverbindung mit der magnetischen Anziehungsbasis (2300) steht, wobei die Antriebsstruktur (3400) elektrisch mit der Anzeige- und Steuerungsbaugruppe (1600) verbunden ist, um das Anheben und Absenken der magnetischen Anziehungsbasis (2300) zu steuern.

4. Pipettierroboter gemäß Anspruch 3, wobei die Probenplatzierungsplatte (2500) mit einer Vielzahl von Probenplatzierungsröhrchen (2502) nebeneinander bereitgestellt ist, wobei die Probenplatzierungslöcher (2501) jeweils innerhalb der Probenplatzierungsröhrchen (2502) ausgebildet sind, und die magnetischen Anziehungselemente (2301) eine Vielzahl von magnetischen Anziehungsstangen (2302) oder magnetischen Anziehungsringen umfassen, die nebeneinander an einem oberen Ende der magnetischen Anziehungsbasis (2300) angeordnet sind, wobei, wenn die magnetische Anziehungsbasis (2300) an der ersten Position ist, jede der magnetischen Anziehungsstangen (2302) an einer Umfangsseite eines jeweiligen der Probenplatzierungsröhrchen (2502) eingeführt ist, oder jeder der magnetischen Anziehungsringe um ein jeweiliges der Probenplatzierungsröhrchen (2502) herum eingerichtet ist.

5. Pipettierroboter gemäß Anspruch 3, wobei die Probenplatzierungsplattenbasis (2200) mit einem Begrenzungsmechanismus bereitgestellt ist, wobei der Begrenzungsmechanismus zum Begrenzen der nach oben gerichteten Bewegung der Probenplatzierungsplatte (2500) während des Anhebens der Magnetanziehungsbasis (2300) in die erste Position konfiguriert ist.

6. Pipettierrobotersteuerungsverfahren unter Verwendung des Pipettierroboters gemäß einem der Ansprüche 1 bis 5, wobei das Pipettierrobotersteuerungsverfahren Folgendes umfasst:
Abrufen von Bildinformationen, die durch die Kamerabaugruppe (1500) gesammelt werden;
Bestimmen gemäß den Bildinformationen von Positionsinformationen für die Reagenzieneinheit, Positionsinformationen für die Probeneinheit, Positionsinformationen für das magnetische Anziehungsmodul und Positionsinformationen für die Probenplatzierungsplatte (2500);
Steuern des Antriebsmechanismus (2400), um die Position der magnetischen Anziehungselemente (2301) anzupassen, um so zu bewirken, dass die magnetischen Anziehungselemente (2301) in die zweite Position abgesenkt werden;
Anpassen einer Position des frei beweglichen Endes und eines Betriebszustands des Plattenbewegungsschlüssels (1410) gemäß den Positionsinformationen für die Probenplatzierungsplatte (2500) und den Positionsinformationen für das magnetische Anziehungsmodul, um die Probenplatzierungsplatte (2500) auf das magnetische Anziehungsmodul zu bewegen;
Anpassen der Position des frei beweglichen Endes und des Betriebszustands der Probenzugabepumpe gemäß den Positionsinformationen für die Probeneinheit und den Positionsinformationen für die Probenplatzierungsplatte (2500), um so zu bewirken, dass die Probenzugabepumpe die zu testende Nukleinsäureprobe aus der Probeneinheit in das Probenplatzierungsloch (2501) der Probenplatzierungsplatte (2500) ansaugt;
Anpassen der Position des sich frei bewegenden Endes und des Betriebszustands der Probenzugabepumpe gemäß den Positionsinformationen für die Reagenzieneinheit und den Positionsinformationen für die Probenplatzierungsplatte (2500), um so zu bewirken, dass die Probenzugabepumpe das Nukleinsäuretesthilfsmaterial von der Reagenzieneinheit in das Probenplatzierungsloch (2501) der Probenplatzierungsplatte (2500) ansaugt, wobei das Nukleinsäuretesthilfsmaterial magnetische Partikel umfasst und sich die magnetischen Partikel mit der zu testenden Nukleinsäureprobe in dem Probenplatzierungsloch (2501) der Probenplatzierungsplatte (2500) mischen;
Steuern, als Reaktion auf ein Mischabschlusssignal, des Antriebsmechanismus (2400), um die Position der magnetischen Anziehungselemente (2301) anzupassen, um so die magnetischen Anziehungselemente (2301) in die erste Position anzuheben, um die magnetischen Partikel in das Probenplatzierungsloch (2501) anzuziehen; und
Anpassen der Position des frei beweglichen Endes und des Betriebszustands der Probenzugabepumpe gemäß den Positionsinformationen für das magnetische Anziehungsmodul, um so eine Flüssigkeit in das Probenplatzierungsloch (2501) anzusaugen, um die Nukleinsäureseparation abzuschließen.

## Revendications

1. Robot de pipetage, caractérisé en comprenant :
un corps d'étanchéité (1100) comprenant un cadre de montage (1110) et une coque d'étanchéité enroulée autour du cadre de montage (1110), ledit cadre de montage (1110) étant muni d'une plate-forme de placement (1120) au bas du cadre de montage (1110), ladite plate-forme de placement (1120) étant munie d'une pluralité de positions de plaque (1130), ladite pluralité de positions de plaque (1130) étant conçues pour au moins placer une unité de réactif, une unité d'échantillon, un module d'attraction magnétique et une plaque de placement d'échantillon (2500) ; ladite unité d'échantillon étant conçue pour placer un échantillon d'acide nucléique à mettre à l'essai, et ladite unité de réactif étant conçue pour placer un matériau auxiliaire d'essai d'acide nucléique, ledit matériau auxiliaire d'essai d'acide nucléique comprenant des particules magnétiques qui sont conçues pour se mélanger à l'échantillon d'acide nucléique à mettre à l'essai ; ladite plaque de placement d'échantillon (2500) comportant une pluralité de trous de placement d'échantillon (2501) ; et ledit module d'attraction magnétique comprenant un mécanisme d'entraînement (2400) et une pluralité d'éléments d'attraction magnétique pouvant être soulevés (2301) entraînés par le mécanisme d'entraînement (2400), et ledit module d'attraction magnétique étant conçu pour placer la plaque de placement d'échantillon (2500) et étant capable de soulever les éléments d'attraction magnétique (2301) vers une première position proche de la plaque de placement d'échantillon (2500) et d'abaisser les éléments d'attraction magnétique (2301) vers une deuxième position éloignée de la plaque de placement d'échantillon (2500) ;
un ensemble de filtration d'air (1200) prévu sur la coque d'étanchéité et conçu pour filtrer et nettoyer l'air entrant et sortant de la coque d'étanchéité ;
un ensemble de mouvement multidirectionnel (1300) prévu sur le cadre de montage (1110), l'ensemble de mouvement multidirectionnel (1300) possédant une extrémité à déplacement libre, ladite extrémité à déplacement libre étant capable d'effectuer un mouvement multidirectionnel au-dessus de la plate-forme de placement (1120) ;
une pompe d'ajout d'échantillon prévue sur l'extrémité à déplacement libre, la pompe d'ajout d'échantillon étant conçue pour injecter et aspirer un liquide ;
une clé de déplacement de plaque (1410) prévue sur l'extrémité à déplacement libre, la clé de déplacement de plaque (1410) étant conçue pour déplacer la plaque de placement d'échantillon (2500) ;
un ensemble caméra (1500) prévu à l'intérieur de la coque d'étanchéité et conçu pour collecter des informations d'image pour l'intérieur de la coque d'étanchéité ; et
un ensemble d'affichage et de commande (1600) raccordé électriquement séparément au mécanisme d'entraînement (2400), à l'ensemble de filtration d'air (1200), à l'ensemble de mouvement multidirectionnel (1300), à la pompe d'ajout d'échantillon, à la clé de déplacement de plaque (1410) et à l'ensemble caméra (1500),
chacune des positions de plaque (1130) étant munie d'une structure de positionnement (3101) sur une face d'extrémité supérieure de la position de plaque (1130), et un espace de montage (3102) étant formé par la structure de positionnement (3101) ; ledit robot de pipetage comprenant en outre un ensemble adaptateur (3000), ledit ensemble adaptateur (3000) étant prévu de manière amovible dans l'espace de montage (3102) ; et au moins l'une de l'unité de réactif, de l'unité d'échantillon, du module d'attraction magnétique et de la plaque de placement d'échantillon (2500) étant prévue sur la position de plaque (1130) par l'ensemble adaptateur (3000) ; et
ledit ensemble adaptateur (3000) comprenant :
une base d'adaptateur (3200) agencée dans l'espace de montage (3102), la structure de positionnement (3101) étant située sur un côté circonférentiel de la base d'adaptateur (3200), et une paroi latérale externe de la base d'adaptateur (3200) étant formée avec au moins une paroi de butée (3201) ;
une structure de butée (3300) prévue de manière mobile sur la base d'adaptateur (3200), durant le mouvement, la structure de butée (3300) possédant une troisième position où la structure de butée (3300) vient en butée fermement contre la structure de positionnement (3101) et amène la paroi de butée (3201) à venir en butée fermement contre la structure de positionnement (3101) pour réaliser le montage et la fixation de la base d'adaptateur (3200), et une quatrième position où la structure de butée (3300) est détachée de la structure de positionnement (3101) pour libérer le montage et la fixation de la base d'adaptateur (3200) ; et
une structure d'entraînement (3400) prévue entre la base d'adaptateur (3200) et la structure de butée (3300) pour commander le mouvement de la structure de butée (3300) ;
ladite base d'adaptateur (3200) étant munie d'une partie de guidage (3202), ladite structure de butée (3300) étant montée de manière coulissante sur la partie de guidage (3202), et ladite partie de guidage (3202) étant munie d'une partie de limitation (3203) au niveau d'une extrémité de queue, ladite partie de limitation (3203) étant munie d'un trou d'évitement (3204), et ladite structure d'entraînement (3400) comprenant une vis d'entraînement (3401), ladite vis d'entraînement (3401) passant à travers le trou d'évitement (3204) et étant raccordée par filetage à la structure de butée (3300), et ladite vis d'entraînement (3401) étant munie d'une tête de vis (3402), ladite tête de vis (3402) étant située sur un côté de la partie de limitation (3203) à l'écart de la structure de butée (3300), et ladite tête de vis (3402) étant d'une taille supérieure à une taille du trou d'évitement (3204).

2. Robot de pipetage selon la revendication 1, ledit robot de pipetage comprenant en outre un dispositif de préhension d'ouverture de couvercle (1420) raccordé électriquement à l'ensemble d'affichage et de commande (1600) ; ladite pompe d'ajout d'échantillon comprenant une pompe d'ajout d'échantillon en ligne (1430) et une pompe d'ajout d'échantillon à sonde unique (1440) ; et ledit ensemble d'affichage et de commande (1600) étant prédéfini avec une pluralité de stratégies de fonctionnement d'essai, chacune des stratégies de fonctionnement d'essai correspondant à un mécanisme de fonctionnement d'essai combiné, ledit mécanisme de fonctionnement d'essai combiné comprenant au moins une combinaison de la pompe d'ajout d'échantillon en ligne (1430) avec la clé de déplacement de plaque (1410), une combinaison de la pompe d'ajout d'échantillon à sonde unique (1440) avec la clé de déplacement de plaque (1410), une combinaison de la pompe d'ajout d'échantillon en ligne (1430) avec le dispositif de préhension d'ouverture de couvercle (1420), une combinaison de la pompe d'ajout d'échantillon à sonde unique (1440) avec le dispositif de préhension d'ouverture de couvercle (1420), et une combinaison de la pompe d'ajout d'échantillon en ligne (1430) avec la pompe d'ajout d'échantillon à sonde unique (1440), et chacune des stratégies de fonctionnement d'essai étant conçue pour commander un mécanisme de fonctionnement d'essai combiné correspondant parmi les mécanismes de fonctionnement d'essai combinés pour terminer un fonctionnement d'essai, et ledit ensemble d'affichage et de commande (1600) étant conçu pour sélectionner l'une correspondante des stratégies de fonctionnement d'essai selon le mécanisme de fonctionnement d'essai combiné.

3. Robot de pipetage selon la revendication 1, ledit module d'attraction magnétique comprenant :
une base de montage (2100) ;
une base (2200) de plaque de placement d'échantillon prévue sur la base de montage (2100), la base (2200) de plaque de placement d'échantillon étant conçue pour monter la plaque de placement d'échantillon (2500) ;
une base d'attraction magnétique (2300) montée de manière à pouvoir être soulevée sur la base de montage (2100) et située sous la base (2200) de plaque de placement d'échantillon, la base d'attraction magnétique (2300) étant munie des éléments d'attraction magnétique (2301), la base d'attraction magnétique (2300) pouvant être élevée à la première position où les éléments d'attraction magnétique (2301) sont proches de la plaque de placement d'échantillon (2500) et être abaissée à la deuxième position où les éléments d'attraction magnétique (2301) sont éloignés de la plaque de placement d'échantillon (2500) ; et
le mécanisme d'entraînement (2400) prévu sur la base de montage (2100) et en liaison de transmission avec la base d'attraction magnétique (2300), la structure d'entraînement (3400) étant raccordée électriquement à l'ensemble d'affichage et de commande (1600) pour commander l'élévation et l'abaissement de la base d'attraction magnétique (2300).

4. Robot de pipetage selon la revendication 3, ladite plaque de placement d'échantillon (2500) étant munie d'une pluralité de tubes de placement d'échantillon (2502) côte à côte, avec les trous de placement d'échantillon (2501) formés dans les tubes de placement d'échantillon (2502) respectivement, et lesdits éléments d'attraction magnétique (2301) comprenant une pluralité de tiges d'attraction magnétique (2302) ou d'anneaux d'attraction magnétique disposés côte à côte sur une extrémité supérieure de la base d'attraction magnétique (2300), lorsque la base d'attraction magnétique (2300) est au niveau de la première position, chacune des tiges d'attraction magnétique (2302) étant insérée sur un côté circonférentiel de l'un respectif des tubes de placement d'échantillon (2502), ou chacun des anneaux d'attraction magnétique étant disposé autour de l'un respectif des tubes de placement d'échantillon (2502).

5. Robot de pipetage selon la revendication 3, ladite base (2200) de plaque de placement d'échantillon étant munie d'un mécanisme de limitation, ledit mécanisme de limitation étant conçu pour limiter un mouvement vers le haut de la plaque de placement d'échantillon (2500) durant l'élévation de la base d'attraction magnétique (2300) à la première position.

6. Procédé de commande de robot de pipetage à l'aide du robot de pipetage selon l'une quelconque des revendications 1 à 5, ledit procédé de commande de robot de pipetage comprenant :
l'acquisition des informations d'image collectées par l'ensemble caméra (1500) ;
la détermination, selon les informations d'image, des informations de position pour l'unité de réactif, des informations de position pour l'unité d'échantillon, des informations de position pour le module d'attraction magnétique et des informations de position pour la plaque de placement d'échantillon (2500) ;
la commande du mécanisme d'entraînement (2400) pour régler la position des éléments d'attraction magnétique (2301) de façon à amener les éléments d'attraction magnétique (2301) à être abaissés à la deuxième position ;
le réglage, selon les informations de position pour la plaque de placement d'échantillon (2500) et les informations de position pour le module d'attraction magnétique, d'une position de l'extrémité à déplacement libre et d'un état de fonctionnement de la clé de déplacement de plaque (1410) de façon à déplacer la plaque de placement d'échantillon (2500) sur le module d'attraction magnétique ;
le réglage, selon les informations de position pour l'unité d'échantillon et les informations de position pour la plaque de placement d'échantillon (2500), de la position de l'extrémité à déplacement libre et de l'état de fonctionnement de la pompe d'ajout d'échantillon de façon à amener la pompe d'ajout d'échantillon à aspirer l'échantillon d'acide nucléique à mettre à l'essai de l'unité d'échantillon dans le trou de placement d'échantillon (2501) de la plaque de placement d'échantillon (2500) ;
le réglage, selon les informations de position pour l'unité de réactif et les informations de position pour la plaque de placement d'échantillon (2500), de la position de l'extrémité à déplacement libre et de l'état de fonctionnement de la pompe d'ajout d'échantillon de façon à amener la pompe d'ajout d'échantillon à aspirer le matériau auxiliaire d'essai d'acide nucléique de l'unité de réactif dans le trou de placement d'échantillon (2501) de la plaque de placement d'échantillon (2500), ledit matériau auxiliaire d'essai d'acide nucléique comprenant des particules magnétiques, et lesdites particules magnétiques se mélangeant à l'échantillon d'acide nucléique à mettre à l'essai dans le trou de placement d'échantillon (2501) de la plaque de placement d'échantillon (2500) ;
la commande, en réponse à un signal de fin de mélange, du mécanisme d'entraînement (2400) pour régler la position des éléments d'attraction magnétique (2301) de façon à élever les éléments d'attraction magnétique (2301) à la première position pour attirer les particules magnétiques dans le trou de placement d'échantillon (2501) ; et
le réglage, selon les informations de position pour le module d'attraction magnétique, de la position de l'extrémité à déplacement libre et de l'état de fonctionnement de la pompe d'ajout d'échantillon de façon à aspirer un liquide dans le trou de placement d'échantillon (2501) pour terminer la séparation d'acide nucléique.
